# EUROPEAN PATENT SPECIFICATION

(11) **EP 4 419 046 B1**
(45) Date of publication and mention of the grant of the patent: **11.11.2026**
(21) Application number: 22873292.1
(22) Date of filing: 21.09.2022
(51) Int. Cl.: A61F 2/24

(54) **A SURGICAL TRICUSPID VALVE PROSTHESIS**
CHIRURGISCHE PROTHESE FÜR DIE TRIKUSPIDALKLAPPE
PROTHÈSE CHIRURGICALE DE VALVULE TRICUSPIDE

(30) Priority: 21.09.2021 SG 10202110444X
(43) Date of publication of application: 28.08.2024
(73) Proprietor: National University of Singapore, Singapore 119077 (SG); National University Hospital (Singapore) Pte Ltd, Singapore 119228 (SG)
(72) Inventor: KOFIDIS, Theodoros, Singapore Yong Loo Lin School of Medicine / Department of Surgery 21 Lower Kent Ridge Road Singapore 119077 (SG)
(74) Representative: Wachinger, Julian Friedrich
(86) International application number: PCT/SG2022/050678
(87) International publication number: WO 2023/048643

(56) References cited:
- WO-A1-2020/214096
- WO-A1-91/19465
- WO-A2-03/037227
- WO-A2-2004/032724
- US-A- 4 960 424
- US-A1- 2014 039 613
- US-A1- 2019 358 034
- US-A1- 2021 169 647

## Description

### CROSS REFERENCE TO RELATED APPLICATIONS

The present application claims the benefit of the Singapore Application No. 10202110444X filed on 21 September 2021.

### TECHNICAL FIELD

The present disclosure relates to a prosthetic heart valve. In particular, the present disclosure relates to a tricuspid valve prosthesis for surgical replacement of a tricuspid valve.

### BACKGROUND

The tricuspid valve is a valve in the heart that is located between the right atrium (upper chamber) and right ventricle (lower chamber). The tricuspid valve allows blood to flow from the right atrium to the right ventricle and ensures that the blood flows in the correct direction from the right atrium to the right ventricle. The tricuspid valve lies within the right trigone of the fibrous skeleton of the heart and consists of the tricuspid valve annulus, the tricuspid valve leaflets, chordae tendineae, and papillary muscles.

The tricuspid valve is generally tri-leaflet, with anterior, septal, and posterior leaflets of unequal sizes. Likewise, there are three papillary muscles: septal/medial papillary muscle, inferior papillary muscle, and anterior papillary muscle. The anterior papillary muscle is the largest and originates from the moderator band as it courses toward the right ventricle free wall. The chordae tendineae connect the papillary muscles to the tricuspid leaflets. The tricuspid valve generally has an area of 7 to 9 cm² and is the largest of the four cardiac valves present in the heart.

The tricuspid valve orifice is made of the tricuspid valve annulus and measures an average of 11.4 cm in males and 10.8 cm in females. The anterosuperior, inferior, and septal margins correspond to each valvular leaflet. Connective tissue around the orifice of the atrioventricular (AV) valves separates the atria from the ventricles, except at the location of the AV bundle. The normal tricuspid annulus is a saddle-shaped structure with the highest points in an antero-posterior orientation and the lowest points in a medio-lateral orientation. In patients with functional tricuspid regurgitation, the annulus dilates along the right ventricular free wall and becomes more circular and planar.

The tricuspid valve has three valve leaflets which are thin and membranous with commissures that appear more like indentations than true commissures. They are each named accordingly for their corresponding positions: anterosuperior, inferior or mural, and septal. In turn, the commissures are named anteroseptal, anteroinferior, and inferior. The anterosuperior leaflet is the largest leaflet. It attaches on the posterolateral aspect of the supraventricular crest and extends from the septal limb to the membranous septum, forming part of the anteroseptal commissure. The posterior leaflet is completely attached to the mural surface, guarding the diaphragmatic surface of the AV junction. It can have multiple scallops and its limits are the inferoseptal and anteroinferior commissures. The septal leaflet is one of the borders of the Triangle of Koch, an anatomical area within the right atrium, with its location demarcated by the coronary sinus orifice, the tendon of Todaro, and the AV node at the apex. Thus, the septal leaflet is a clinical landmark to aid in the localization of this conductive tissue during surgery of the right atrioventricular valve.

The chordae tendineae are thin strong inelastic fibrous cords that extend from the free edge of the cusps of the tricuspid valve to the apices of the papillary muscles within the right ventricle. They transmit the force of the contracting papillary muscles during ventricular systole to the cusps of the valves to prevent the valves from prolapsing into the atria due to the higher pressures generated in the ventricle.

There are three papillary muscles in the RV: anterior, inferior, and medial or septal; where anterior papillary muscle is the largest and others are small muscular tips. Each of these muscles correspond to the valvular leaflets it supports in both size and location. The anterior papillary muscle arises from the right anterolateral ventricular wall (TV), below the anteroinferior commissure of the inferior leaflet, blending with the right end of the septomarginal trabecula (SMT).

Contrary to its nomenclature, the tricuspid valve (TV) behaves like a bicuspid valve, in that the septal leaflet remains fixed between the right and left fibrous trigones and the atrial and ventricular septa, while the anterior and posterior leaflets fan into the RV during diastole, and fan back to seal the valve during systole. The annulus helps in this by dilating and constricting during diastole and systole, respectively.

In some people, this valve does not function correctly, and they are said to have tricuspid valve disease. Tricuspid valve disease is rare compared with other types of valve disease. The most common form of tricuspid valve disease is tricuspid stenosis, which means the valve leaflets are stiff and do not open all the way. This makes the valve narrow and restricts the blood flow. Another form of valve disease is tricuspid regurgitation. Patients with this condition have leaflets that do not close all the way, and blood leaks backward across the valve instead of flowing into the ventricle. These conditions cause the heart to pump harder to move blood through the body.

There are many surgical treatment options for tricuspid valve disease, one of which is the replacement of the tricuspid valve with a prosthetic valve. Tricuspid valve replacement surgery may be done using traditional open-heart surgery or minimally invasive methods, which involve smaller incisions than those used in open-heart surgery and may include robot-assisted techniques. Existing replacement valves fall into two categories: tissue valves, and mechanical valves. Tissue or biological valves are made from animal tissues and valves, with valve leaflets that are soft and thin. Biological tissue valves eventually need to be replaced as they degenerate over time. Biological valves require short-term use of blood-thinning medicines for about three to six months. These medications can usually be discontinued at that time unless there is another medical reason to continue use, such as irregular heartbeats. On the other hand, mechanical valves made from carbon fibers have solid valve leaflets. Patients who have undergone a valve replacement will need to follow antibiotic prophylaxis throughout their lifetime. Patients who have received a mechanical valve will need life-long treatment with coumadin. This medication thins the blood to prevent catastrophic clots from forming on the valve leaflets themselves. Furthermore, present replacement valves are only connected to the heart at the tricuspid valve annulus and do not interact or "cross-talk" with the right ventricle. This lack of interaction with the right ventricle may lead to heart failure in the recipient of the replacement valve.

Therefore, existing replacement valves have shortcomings as they are shaped unnaturally, contain bulky foreign material, require strong anticoagulation, do not last long, and do not help the heart recover efficiently. There is thus a need for a tricuspid valve prosthesis that avoids these issues of existing replacement valves. US4960424A discloses a replacement atrio-ventricular valve from a donor heart that includes an annulus, a plurality of chordae, and papillary muscles attached thereto.

### SUMMARY

The invention is a tricuspid valve prosthesis as defined in claim 1. There is provided according to some embodiments of the disclosure, a tricuspid valve prosthesis to be transplanted into a heart, the tricuspid valve prosthesis comprising: two or more leaflets each comprising an annular length and two free edges forming a tip, the two or more leaflets joined together at commissures to create a ring, and configured to coapt with each other; and a connector element connected to the tips of the two or more leaflets. Optionally, the two or more leaflets may coapt with each other edge-to-edge. Optionally, the two or more leaflets may coapt with each other surface-to-surface.

According to some embodiments, the connector element may be configured to be attached to a papillary muscle of the heart. Optionally, the papillary muscle may be the anterior papillary muscle.

According to some embodiments, the connector element may be configured to be attached to a free wall of the right ventricle.

According to some embodiments, the tricuspid valve prosthesis may further comprise two or more sets of cords, each set of cords attached to the connector element at a first end.

According to some embodiments, the tricuspid valve prosthesis may further comprise two or more sets of cords, each set of cords attached to the tip of one of the two or more leaflets at a first end, the two or more sets of cords surrounded by the connector element. Optionally, the connector element may surround a first end of the two or more sets of cords. Optionally, the connector element spans the length of the two or more sets of cords. Optionally, the two or more sets of cords may be configured to be attached to one or more papillary muscles of the heart at a second end.

According to some embodiments, the ring may be bean-shaped or saddle-shaped.

According to some embodiments, the free edges may be shaped along a concave arc of a circle.

According to some embodiments, the two or more leaflets may further comprise a slot that splits a tip of the two or more leaflets, the slot comprising two circular arcs. Optionally, the two circular arcs may be configured to coapt with each other.

There is further provided according to some embodiments of the disclosure, a tricuspid valve prosthesis to be transplanted into a heart, the tricuspid valve prosthesis comprising: two or more leaflets each comprising an annular length and two free edges forming a tip, the two or more leaflets joined together at commissures to create a ring, and configured to coapt with each other; and two or more sets of cords, each set of cords attached to the two or more leaflets at a first end. Optionally, the two or more sets of cords may be sutured together to form a cap, the cap configured to be attached to a papillary muscle of the heart. Optionally, the papillary muscle is the anterior papillary muscle.

According to some embodiments, the two or more sets of cords may be sutured together to form a cap, the cap configured to be attached to a free wall of the right ventricle.

According to some embodiments, the ring may be bean-shaped or saddle-shaped.

According to some embodiments, the free edges may be shaped along a concave arc of a circle.

According to some embodiments, the two or more leaflets may further comprise a slot that splits a tip of the two or more leaflets, the slot comprising two circular arcs. Optionally, the two circular arcs may be configured to coapt with each other.

According to some embodiments, the tricuspid valve prosthesis disclosed herein, further comprising a stent frame, wherein two or more leaflets may be positioned within the stent frame and wherein the ring may be dimensioned to match perimeter of the stent frame.

According to some embodiments, the stent frame may comprise one or more slots for inserting the two or more leaflets to the stent frame.

According to some embodiments, the stent frame may comprise a plurality of holes for stitching of the two or more leaflets.

According to some embodiments, the stent frame may comprise a medical grade material, for example stainless steel, silicon, plastic, graphene oxide, or mixture thereof.

According to some embodiments, the tricuspid valve prosthesis disclosed herein may further comprise a cuff attached to the stent frame. Optionally, the cuff may be coated with one or more drugs.

### BRIEF DESCRIPTION OF THE DRAWINGS

In order for the present disclosure, to be better understood and for its practical applications to be appreciated, the following Figures are provided and referenced hereafter. It should be noted that the Figures are given as examples only and in no way limit the scope of the invention.
Fig. 1 is a schematic illustration of a tricuspid valve prosthesis, in accordance with embodiments of the present disclosure;
Fig. 2 is a schematic illustration of a tricuspid valve prosthesis implanted in a heart with cords attached to papillary muscles, in accordance with embodiments of the present disclosure;
Fig. 3A is a schematic illustration of a tricuspid valve prosthesis, which is an alternative embodiment of the tricuspid valve prosthesis of Fig. 1, with cords stitched together, in accordance with embodiments of the present disclosure;
Fig. 3B is a schematic illustration of a tricuspid valve prosthesis, which is an alternative embodiment of the tricuspid valve prosthesis of Fig. 1, with cords secured to a connector element, in accordance with embodiments of the present disclosure;
Fig. 3C is a schematic illustration of a tricuspid valve prosthesis, which is another alternative embodiment of the tricuspid valve prosthesis of Fig. 1, with a connector element, in accordance with embodiments of the present disclosure;
Figs. 4A and 4B are schematic illustrations of methods of implanting a tricuspid valve prosthesis into a heart, in accordance with embodiments of the present disclosure;
Fig. 5A is a schematic illustration of a side perspective view of leaflets and ring of a first alternative tricuspid valve prosthesis and Fig. 5B is a schematic illustration of a bottom view of leaflets of a first alternative tricuspid valve prosthesis, in accordance with embodiments of the present disclosure;
Fig. 5C is a schematic illustration of a leaflet and cord of first alternative tricuspid valve prosthesis, in accordance with embodiments of the present disclosure;
Fig. 5D is a schematic illustration of a top view of results of a finite element method (FEM) analysis of leaflets of first alternative tricuspid valve prosthesis under external pressure of 23 mmHg and Fig. 5E is a schematic illustration of a side view of results of a finite element method (FEM) analysis of leaflets of first alternative tricuspid valve prosthesis under maximum principal stress (MPa), in accordance with embodiments of the present disclosure;
Fig. 6A is a schematic illustration of a side perspective view of a second alternative tricuspid valve prosthesis, in accordance with embodiments of the present disclosure;
Fig. 6B is a schematic illustration of second alternative tricuspid valve prosthesis in an open state, and Fig. 6C is a schematic illustration of second alternative tricuspid valve prosthesis in a closed state, in accordance with embodiments of the present disclosure;
Fig. 6D is a schematic illustration of an alternative cord connected to a leaflet of second alternative tricuspid valve prosthesis, in accordance with embodiments of the present disclosure;
Fig. 7A is a schematic illustration of leaflets of a third alternative tricuspid valve prosthesis, in accordance with embodiments of the present disclosure;
Fig. 7B is a schematic illustration of a leaflet of third alternative tricuspid valve prosthesis, in accordance with embodiments of the present disclosure;
Fig. 7C is a schematic illustration of a top view of results of a finite element method (FEM) analysis of leaflets of third alternative tricuspid valve prosthesis under external pressure of 23 mmHg, in accordance with embodiments of the present disclosure;
Fig. 8A is a schematic illustration of leaflets of a fourth alternative tricuspid valve prosthesis, in accordance with embodiments of the present disclosure;
Fig. 8B is a schematic illustration of a top view of results of a finite element method (FEM) analysis of leaflets of fourth alternative tricuspid valve prosthesis under external pressure of 23 mmHg, in accordance with embodiments of the present disclosure;
Fig. 9A is a schematic illustration of a side perspective view of a fifth alternative tricuspid valve prosthesis designed for a type I tricuspid valve and Fig. 9B is a schematic illustration of a top view of leaflets of fifth alternative tricuspid valve prosthesis designed for a type I tricuspid valve, in accordance with embodiments of the present disclosure;
Fig. 9C is a schematic illustration of leaflets and cords of fifth alternative tricuspid valve prosthesis, in accordance with embodiments of the present disclosure;
Fig. 9D is a schematic illustration of a top view of results of a finite element method (FEM) analysis of leaflets of fifth alternative tricuspid valve prosthesis under external pressure of 23 mmHg, in accordance with embodiments of the present disclosure;
Fig. 10A is a schematic illustration of a side perspective view of a sixth alternative tricuspid valve prosthesis designed for a type IIIB tricuspid valve and Fig. 10B is a schematic illustration of a top view of leaflets of sixth alternative tricuspid valve prosthesis designed for a type IIIB tricuspid valve, in accordance with embodiments of the present disclosure;
Fig. 10C is a schematic illustration of leaflets and cords of sixth alternative tricuspid valve prosthesis, in accordance with embodiments of the present disclosure;
Fig. 10D is a schematic illustration of a top view of results of a finite element method (FEM) analysis of leaflets of sixth alternative tricuspid valve prosthesis under external pressure of 23 mmHg, in accordance with embodiments of the present disclosure;
Fig. 11A is a schematic illustration of a side perspective view of a seventh alternative tricuspid valve prosthesis and Fig. 11B is a schematic illustration of a top view of leaflets of seventh alternative tricuspid valve prosthesis, in accordance with embodiments of the present disclosure;
Fig. 11C is a schematic illustration of leaflets and cords of seventh alternative tricuspid valve prosthesis, in accordance with embodiments of the present disclosure; and
Fig. 11D is a schematic illustration of a top view of results of a finite element method (FEM) analysis of leaflets of seventh alternative tricuspid valve prosthesis under external pressure of 23 mmHg, in accordance with embodiments of the present disclosure.
Fig. 12A is a schematic illustration of a top view of a stent comprised in the tricuspid valve prosthesis, in accordance with some embodiments of the present disclosure;
Fig. 12B is a schematic illustration of a first side perspective view of the stent comprised in the tricuspid valve prosthesis, in accordance with some embodiments of the present disclosure;
Fig. 12C is a schematic illustration of a second side perspective view of the stent comprised in the tricuspid valve prosthesis, in accordance with some embodiments of the present disclosure;
Fig. 12D is a schematic illustration of a third side perspective view of the stent comprised in the tricuspid valve prosthesis, in accordance with some embodiments of the present disclosure;
Fig. 13A is a schematic illustration of a side perspective view of the stented tricuspid valve prosthesis, in accordance with some embodiments of the present disclosure;
Fig. 13B is a schematic illustration of a top view of the stented tricuspid valve prosthesis, in accordance with some embodiments of the present disclosure;
Figs. 13C is a schematic illustration of an anterior leaflet for the stented tricuspid valve prosthesis, in accordance with some embodiments of the present disclosure;
Figs. 13D is a schematic illustration of a posterior leaflet of the stented tricuspid valve prosthesis, in accordance with some embodiments of the present disclosure;
Figs. 13E is a schematic illustration of a septal leaflet of the stented tricuspid valve prosthesis, in accordance with some embodiments of the present disclosure;
Fig. 14A is an image of a top view of the stented tricuspid valve prosthesis, in accordance with some embodiments of the present disclosure;
Fig. 14B is an image of a side perspective view of the stented tricuspid valve prosthesis, in accordance with some embodiments of the present disclosure;
Figs. 15A is an image of a top view of the stented tricuspid valve prosthesis during the valve closing, in accordance with some embodiments of the present disclosure; and
Figs. 15B is an image of a top view of the stented tricuspid valve prosthesis during the valve opening, in accordance with some embodiments of the present disclosure.

Identical or duplicate or equivalent or similar structures, elements, or parts that appear in one or more drawings are generally labelled with the same reference numeral, optionally with an additional letter or letters to distinguish between similar entities or variants of entities and may not be repeatedly labelled and/or described. References to previously presented elements are implied without necessarily further citing the drawing or description in which they appear.

### DETAILED DESCRIPTION

In the following detailed description, numerous specific details are set forth in order to provide a thorough understanding of the invention. However, it will be understood by those of ordinary skill in the art that the invention may be practiced without these specific details. In other instances, well-known methods, procedures, components, modules, units and/or circuits have not been described in detail so as not to obscure the invention.

Dimensions of components and features shown in the figures are chosen for convenience or clarity of presentation and are not necessarily shown to scale or true perspective. For convenience or clarity, some elements or structures are not shown or shown only partially and/or with different perspective or from different point of views.

Although embodiments of the invention are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, some of the described method embodiments or elements thereof can occur or be performed simultaneously, at the same point in time, or concurrently. Unless otherwise indicated, use of the conjunction "or" as used herein is to be understood as inclusive (any or all of the stated options).

With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.

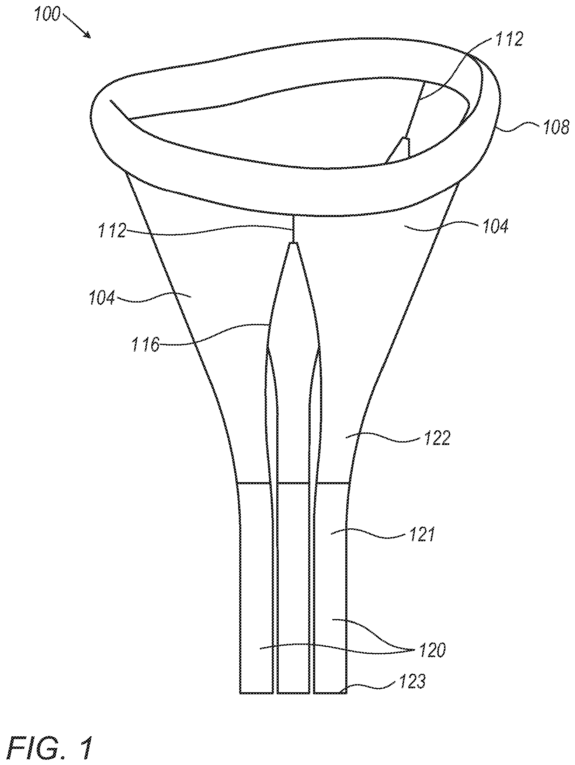

Fig. 1 is a schematic illustration of a tricuspid valve prosthesis 100, in accordance with embodiments of the present disclosure. Tricuspid valve prosthesis 100 may have features that are similar to a natural human tricuspid valve. Tricuspid valve prosthesis 100 is meant to closely reflect the function of the native tricuspid valve, which moves with the natural distortion of the heart muscle. The tricuspid valve prosthesis 100 includes two or more flexible, membrane-like leaflets 104 connected to each other to form an orifice, annulus, or ring 108 for attachment to a tricuspid valve annulus in the heart to allow blood to flow through in one direction. Ring 108 mimics the native tricuspid valve annulus and may be a flat bean-shaped ring, flat circular ring, or a saddle-shaped ring. In some embodiments, ring 108 may be flexible or rigid. In some embodiments, ring 108 may be formed with an annular length (not shown) of leaflets 104. In some embodiments, ring 108 may be formed from, for example, an elastic annuloplasty ring. In some embodiments, ring 108 may be formed by folding or rolling leaflets 104 onto itself towards the outer side of tricuspid valve prosthesis 100. In some embodiments, ring 108 may be further strengthened by the addition of strips of suitable material such as biomedical fibres, polymers, or bovine pericardium. Rolling or folding leaflets 104 onto itself towards the outer side of tricuspid valve prosthesis 100 may advantageously assist in avoiding the creation of clots at the inner side of tricuspid valve prosthesis 100, and if clots are to be created, they would only appear on the outer side of tricuspid valve prosthesis 100 at the area of the fold or roll of the ring 108 or ring portion, which poses less risk of damaging the efficient operation of tricuspid valve prosthesis 100. In some embodiments, the rolling or folding of leaflets 104 forming ring 108 does not include stitching. In some embodiments, the flexible ring 108 may also be termed as "non-stented ring".

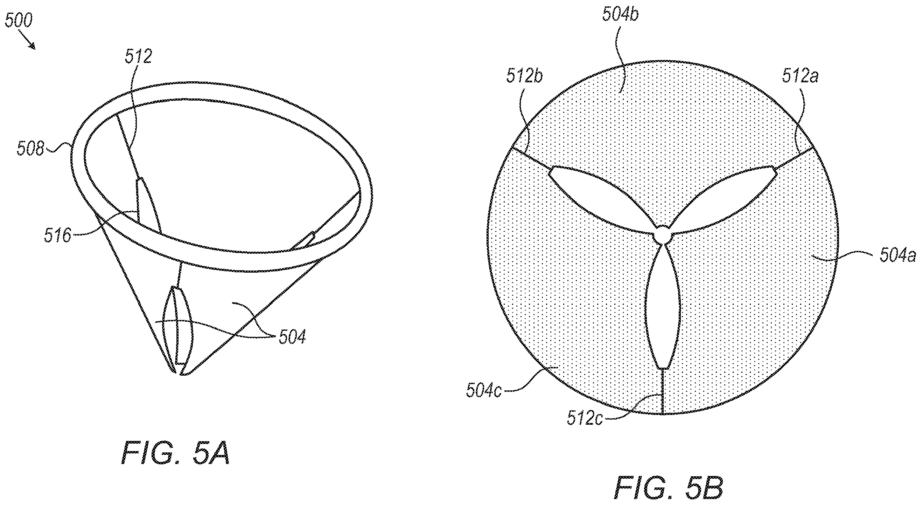

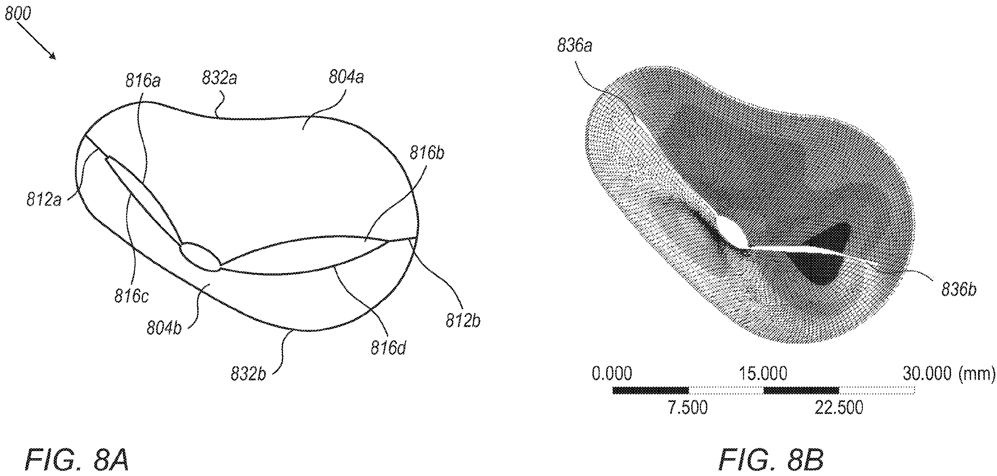

In some embodiments of the present disclosure, leaflets 104 mimic the function of native tricuspid valve leaflets. In some embodiments, each leaflet 104 may mimic the function of one or more native tricuspid valve leaflets. In some embodiments, two or more leaflets 104 may mimic the function of one native tricuspid valve leaflet. In some embodiments, there may be a first leaflet mimicking the function of a patient's native posterior leaflet, a second leaflet mimicking the function of a patient's native septal leaflet, and a third leaflet mimicking the function of a patient's native anterior leaflet (see Figs. 5A-5E, 6A-6D, 7A-7C, 9A-9D, 10A-10D, and 11A-11D). In some embodiments, there may be a first leaflet and second leaflet mimicking the function of a patient's native posterior leaflet, a third leaflet and fourth leaflet mimicking the function of a patient's native septal leaflet, and a fifth leaflet and sixth leaflet mimicking the function of a patient's native anterior leaflet (not shown). In some embodiments, there may be a first leaflet mimicking the function of a patient's native septal leaflet, and a second leaflet mimicking the function of a patient's native posterior leaflet and anterior leaflet (see Figs. 8A-8B). The number, dimensions, shape, and orientation of leaflets 104 may be customised and adjusted based on the size, shape and morphology type of a patient's native tricuspid valve and annulus, as well as the position of a patient's native papillary muscles. In some embodiments, leaflets 104 may be customized and designed based on images of the patient's native tricuspid valve. The images may be obtained through an echocardiography study (or other imaging study). From the imaging study, heart chamber sizes and movements are measured. The detailed dimensions of the patient's tricuspid valve annulus, leaflets and cords are also measured from the acquired images. A three-dimensional echocardiography study can be performed with, for example, a transesophageal echocardiography (TEE) probe or a transthoracic echocardiography (TTE) probe. Segments of the tricuspid valve can be three-dimensionally and four-dimensionally modelled and measured using software such as eSieValves.TM. (Siemens Medical Solutions USA, Inc., Malvern, Pa.). Relevant measurements can include outer and inner diameters of the annulus, annular areas, intertrigonal and intercommisure distances, lengths along various axes of the anterior, septal, and posterior leaflets, and locations of papillary muscles. In addition, or alternatively, a three-dimensional study of a tricuspid valve can be performed with computed tomography (CT) or magnetic resonance imaging (MRI). Segmentation of the tricuspid valve area can be performed using the image analysis software and relevant measurements can be obtained. After leaflets 104 have been designed, the dimensions of leaflets 104 may be optimized with finite element method (FEM) analysis to look at how leaflets 104 may coapt, deform and perform under external pressure within the heart.

In some embodiments of the present disclosure, leaflets 104 may be formed from natural material or biocompatible composite material which can resist clotting. In some embodiments, adjacent leaflets 104 may be connected to each other at two ends of the annular length (not shown) to form commissures 112. In some embodiments, leaflets 104 may be made of separate pieces of material and adjacent pairs of leaflets 104 sutured together at commissure 112. In other embodiments, leaflets 104 may be constructed from a single piece of material. In some embodiments, commissures 112 may be labelled with laser or with any other appropriate method to assist with orientating tricuspid valve prosthesis 100 during implantation.

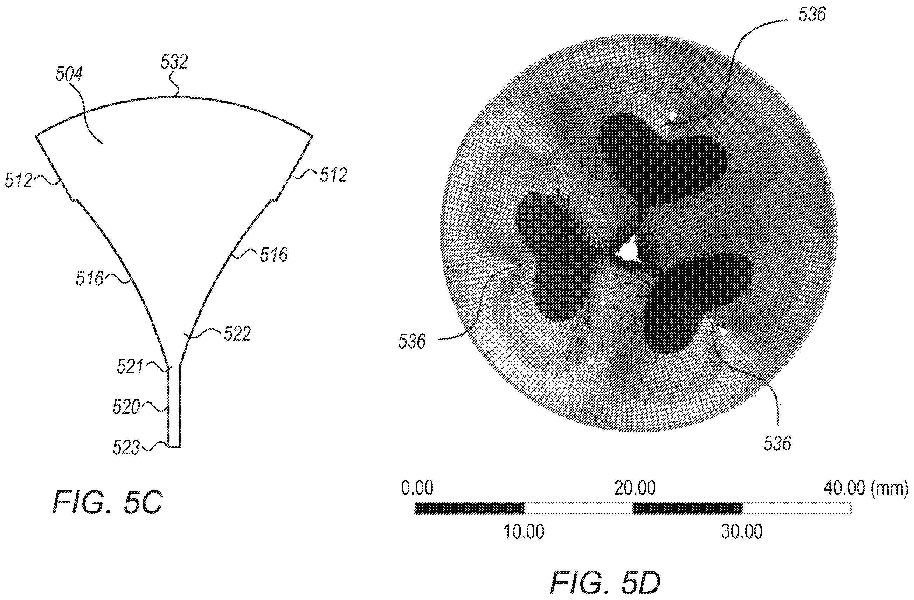

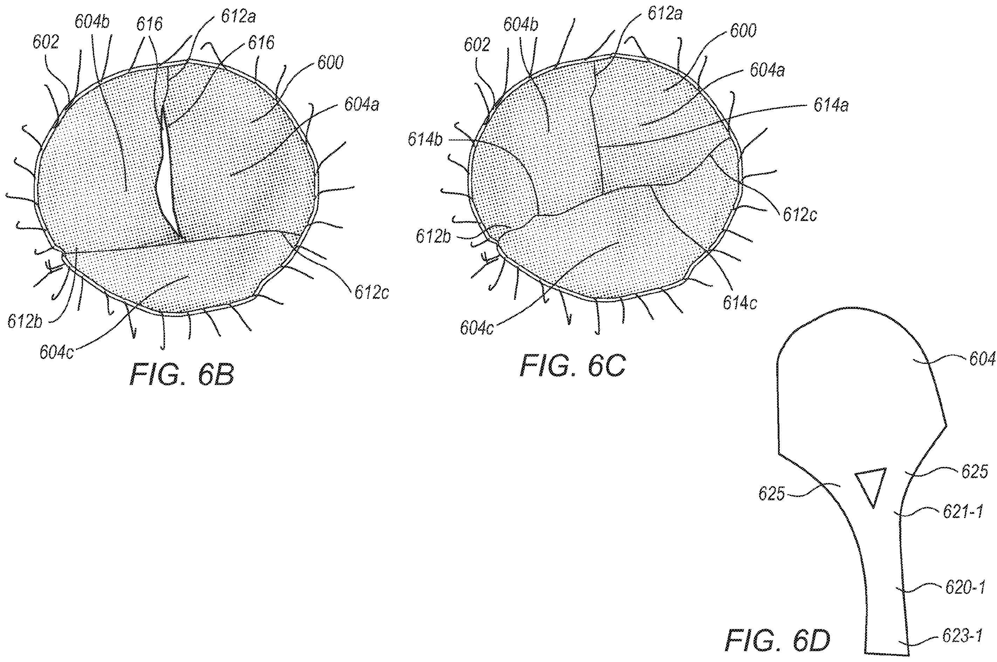

In some embodiments of the present disclosure, leaflets 104 may generally be shaped as a three-sided shape, comprising an annular length (not shown) at a top and two free edges 116 forming a tip 122. In some embodiments, free edges 116 may taper towards each other forming tip 122. Free edges 116 may be shaped in the form of concave arcs. In some embodiments, in a closed state, free edges 116 of a leaflet 104 may coapt edge-to-edge, whereby free edges 116 of a leaflet may coapt or meet with neighbouring free edges 116 located within the same leaflet or a neighbouring leaflet 104 forming a coaptation edge (not shown) to seal and close tricuspid valve prosthesis 100 (see Figs. 5D, 5E, 7C, 8B, 9D, 10D, and 11D). This differs from a patient's native tricuspid valve where the leaflets open and close by contacting each other or coapting surface-to-surface. In some embodiments, in a closed state, there may be increased coaptation between leaflets 104 such that in addition to free edges 116, leaflets 104 may coapt surface-to-surface with a neighbouring leaflet 104 (see Fig. 6C). In an open state, free edges 116 of a leaflet 104 do not contact each other. In some embodiments, tricuspid valve prosthesis 100 may be biased to a closed position. In other embodiments, tricuspid valve prosthesis 100 may be biased to an open position.

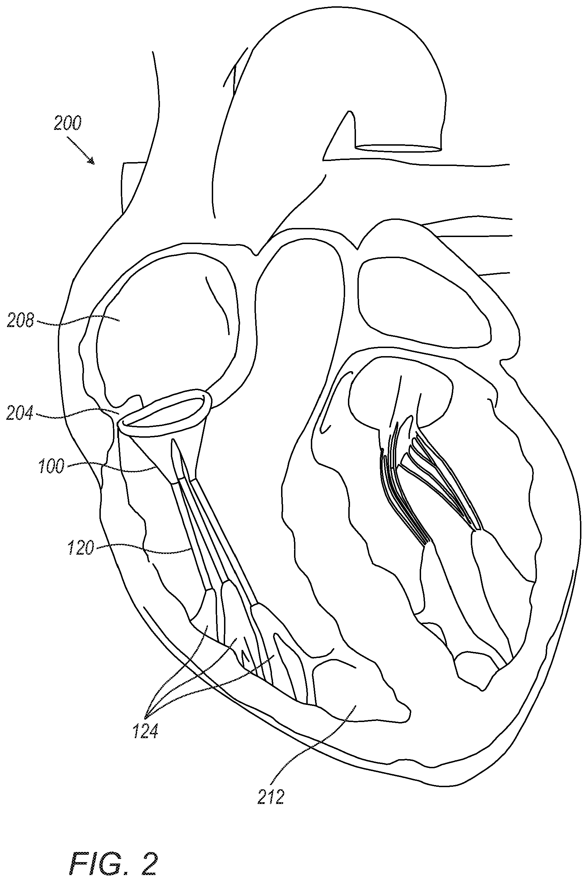

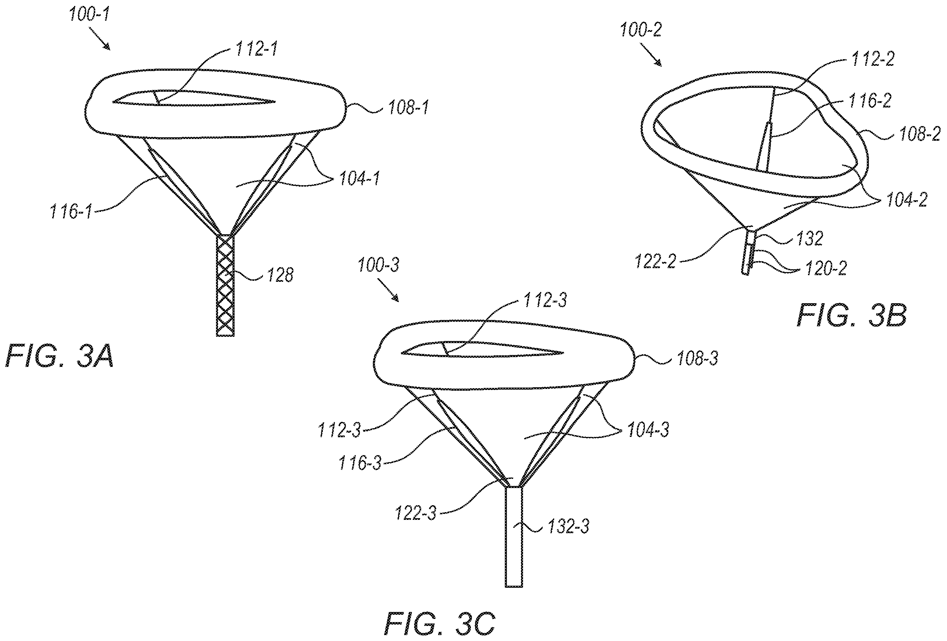

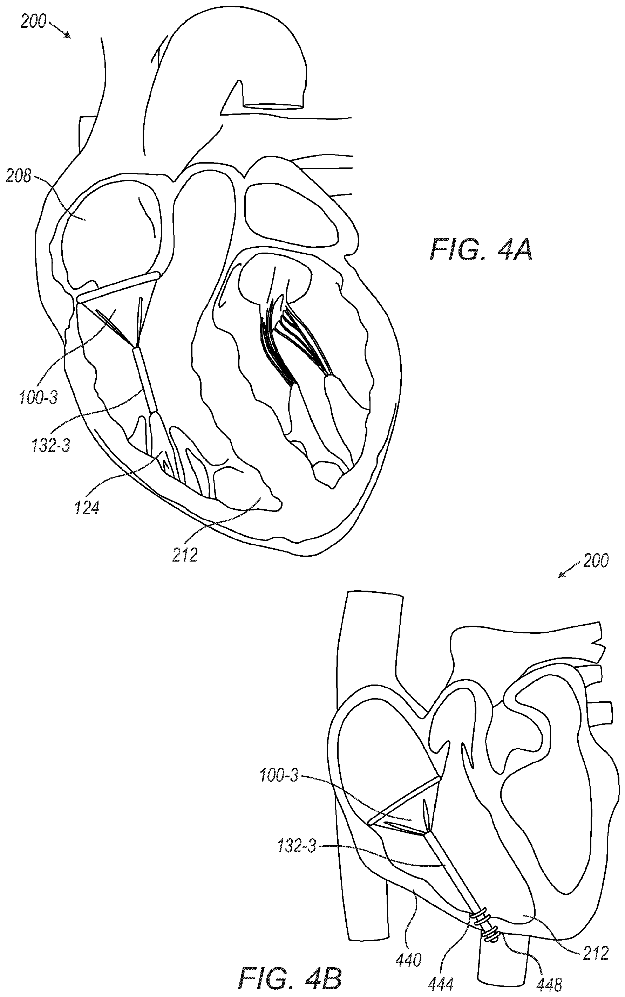

In some embodiments of the present disclosure, the tricuspid valve prosthesis 100 may further comprise cords 120 which mimic the native chordae tendineae of the heart. Each cord 120 may comprise a first end 121 and a second end 123. Each cord 120 may be connected at first end 121 to a tip 122 or a body of leaflet 104. In other embodiments, the tip 122 of leaflet 104 is connected to a connector element (such as connector element 132 of Fig. 3B). In this embodiment, the connector element 132 may be connected to the tips 122 of all the leaflets 104. In some embodiments, the connector element 132 is connected at its distal end to first end 121 of cord 120. In some embodiments, there may be one or more cords 120 connected to each leaflet 104. In some embodiments, first end 121 of cord 120 may split into branches, with each branch connected to leaflet 104 (see Fig. 6D). Each cord 120 may be formed from the same piece of material as leaflet 104 or may be formed from a separate piece of material and then attached to leaflet 104. In some embodiments, cords 120 may be connected at second end 123 to one or more papillary muscles of the right ventricle (see Figs. 2 and 4A) or connected to a free wall of the right ventricle (see Fig. 4B). Cords 120 may be connected to the same or different papillary muscle. In some embodiments, where there are three leaflets 104, each of which has one cord 120, each of the cords 120 may be connected to one of the three papillary muscles of the right ventricle (see Fig, 2). In other embodiments, cords 120 may all be connected to one papillary muscle of the right ventricle, preferably the anterior papillary muscle which is the strongest papillary muscle of the right ventricle. In other embodiments, second ends 123 of cords 120 may be connected to a free wall of a right ventricle (see Fig. 4B). Cords 120 connecting leaflets 104 to the papillary muscles or free wall of the right ventricle of the heart allow tricuspid valve prosthesis 100 to interact or "cross-talk" with the heart ventricle, thus reducing the risk of heart failure after implantation.

In some embodiments of the present disclosure, any or all components of tricuspid valve prosthesis 100, including ring 108, leaflets 104, cords 120 and connector element 132 (see Figs. 3B and 3C) may be produced with natural materials and can avoid the inclusion of foreign material, such as pledgets. Homograft material and/or composite material, including various combinations of homograft, xenograft and/or autograft material, may be used to fabricate the ring 108, leaflets 104, cords 120, and connector element 132 (see Figs. 3B and 3C). The material used to form any component of tricuspid valve prosthesis 100 may include, but is not limited to, human, bovine or porcine pericardium, decellularized bioprosthetic material, polymers including natural and synthetic polymers, woven biodegradable polymers incorporated with cells, and extracellular materials. Biodegradable natural polymers can include, but are not limited to fibrin, collagen, chitosan, gelatin, hyaluronan, and similar materials thereof. A biodegradable synthetic polymer scaffold that can be infiltrated with cells and extracellular matrix materials can include, but is not limited to, poly(L-lactide), polyglycolide, poly(lactic-co-glycolic acid), poly(caprolactone), polyorthoesters, poly(dioxanone), poly(anhydrides), poly(trimethylene carbonate), polyphosphazenes, and similar materials thereof. Flexible rings 108 may be further customized to provide individualized flexibility or rigidity for the patient. Additionally, any component of the tricuspid valve prosthesis 100 may be fashioned intraoperatively with autologous pericardium of the patient. In some embodiments, a tricuspid valve prosthesis 100 can be fabricated from the patient's own pericardium. Alternatively, the tricuspid valve prosthesis 100 may be fabricated from xenogeneic materials (e.g., animal tissues, such as existing valves) over which a layer of the patient's own cultured cells is applied by means of tissue engineering. **In** other embodiments, any or all components of tricuspid valve prosthesis 100, including ring 108, leaflets 104, cords 120 and connector element 132 (see Figs. 3B and 3C) may be made of synthetic or non-biodegradable materials like plastics, silicones, or stainless steel.

Artificial valves are frequently fixed with glutaraldehyde, which is a known toxin and promotes regeneration. Tricuspid valve prostheses of the present invention may be fixed by non-glutaraldehyde-based methods, such as dye-mediated photofixation. Tricuspid valve prostheses of the present disclosure may also be fixed by using alternative cross-linking agents, such as epoxy compounds, carbodiimide, diglycidyl, reuterin, genipin, diphenylphosphorylazide, acyl azides, and cyanamide, or by physical methods, such as ultraviolet light and dehydration.

In some embodiments of the present disclosure, tricuspid valve prosthesis 100, or some components of tricuspid valve prosthesis 100, may be produced directly with biological three-dimensional (3D) printing using biological materials. In other embodiments, tricuspid valve prosthesis 100, or some components of tricuspid valve prosthesis 100, may be produced using a template or mould constructed by three-dimensional printing, based on the detailed dimensions obtained from three-dimensional imaging performed pre-operatively.

Fig. 2 is a schematic illustration of a tricuspid valve prosthesis 100 implanted in a heart 200 with cords 120 attached to papillary muscles 124, in accordance with embodiments of the present disclosure. The tricuspid valve prosthesis 100 is shown implanted at the location of the native tricuspid valve annulus 204 located between the right atrium 208 and the right ventricle 212. Cords 120 of tricuspid valve prosthesis 100 are shown connected to papillary muscles 124. Cords 120, tethering leaflets 104 (not shown) to the papillary muscles 124 of the patient, advantageously provide support to the right ventricular wall throughout the cardiac cycle and prevent the leaflets 104 from opening into the right atrium 208. In some embodiments, cords 120 may be between 5 and 15 mm in length, and between 1 and 5 mm in width. In some embodiments, the lengths and widths of cords 120 are adjustable and may be determined based on preoperative echo scans and CT scans performed on the patient receiving tricuspid valve prosthesis 100. In some embodiments, the length of cords 120 may be determined by measuring the distance from a native tricuspid valve annulus 204 to the tip of papillary muscles 124.

In some embodiments of the present disclosure, ring 108 may be custom-made following an ultrasound examination of a patient's heart. In particular, a three-dimensional echocardiography study may be performed to obtain detailed anatomical measurements and/or render a three-dimensional model of the patient's heart from which a customized tricuspid valve prosthesis can be produced. Leaflets 104 and cords 120 may also be customized based upon ultrasound imaging of the subject's native tricuspid valve and surrounding anatomy. In some embodiments, customized tricuspid valve prostheses may also be produced from data obtained by other imaging modalities which provide three-dimensional information, including cardiac CT and cardiac MRI. Thus, tricuspid valve prostheses of the present disclosure may be selected, customized or designed to match the patient's specific anatomy.

In some embodiments of the present disclosure, prior to removal or explantation of the native tricuspid valve, the native commissure positions may be marked with a double-armed suture. After the native tricuspid valve has been removed, the sutures marking the native commissure positions may be passed through corresponding points on ring 108 of tricuspid valve prosthesis 100 to guide tricuspid valve prosthesis 100 into the heart 200, such that the corresponding points on ring 108 will land on the double-armed suture marked native commissure positions. In some embodiments, the corresponding points on tricuspid valve prosthesis 100 may be commissures 112. Once ring 108 is positioned within the native tricuspid valve annulus 204, the commissural sutures may be secured by tying. Cords 120 may then be secured onto their corresponding papillary muscle(s). In some embodiments, cords 120 may be attached to their corresponding papillary muscle(s) prior to orientation of ring 108 of tricuspid valve prosthesis 100.

Fig. 3A is a schematic illustration of a tricuspid valve prosthesis 100-1, which is an alternative embodiment of the tricuspid valve prosthesis 100 of Fig. 1, with cords 120 stitched together, in accordance with embodiments of the present disclosure. The embodiment of Fig. 3A is similar to the embodiment of Fig. 1, with like-numbered terms configured in the same way, except that the cords 120-1 are stitched together length-wise to form a cap 128. Cap 128 may then be stitched and secured to a papillary muscle 124 of a patient, preferably the anterior papillary muscle (see Fig. 4A). In other embodiments, cap 128 may be secured to a free wall 440 of the right ventricle 212 (see Fig. 4B). Cap 128 ensures a single vector of pulling when the heart contracts. Cap 128 also advantageously strengthens the connection between leaflets 104-1 and the papillary muscle 124 or the free wall 440 of the right ventricle 212 as cap 128 brings cords 120-1 together to prevent tearing of cords 120-1 when the papillary muscle 124 or free wall 440 of the right ventricle 212 pulls on cords 120-1 during heart contraction. Cap 128 also ensures uniformity during coaptation so that the coaptation of free edges 116-1 of tricuspid valve prothesis 100-1 is not compromised during heart contraction.

In other embodiments, cords 120-1 may be stitched together length-wise over a portion of cords 120-1 at a first end connected to leaflets 104-1 such that cords 120-1 are free from each other at a second end and may be attached to separate papillary muscles (not shown). The stitching ensures that there is a single vector of pulling when the heart 200 contracts, even though cords 120-1 may be connected to different papillary muscles. In some embodiments, cords 120-1 may be between 2 and 10 mm in length and may be stitched lengthwise over a length of between 2 and 10 mm. In some embodiments, the lengths and widths of cords 120-1, as well as the length over which cords 120-1 are stitched are adjustable and may be determined by preoperative echo scans and CT scans performed on the patient receiving tricuspid valve prosthesis 100-1.

Fig. 3B is a schematic illustration of a tricuspid valve prosthesis 100-2, which is an alternative embodiment of the tricuspid valve prosthesis 100 of Fig. 1, with cords 120-2 secured to a connector element 132, in accordance with embodiments of the present disclosure. The embodiment of Fig. 3B is similar to the embodiment of Fig. 1, with like-numbered terms configured in the same way, except that cords 120-2 are secured to a connector element 132 at a first end of cords 120-2 joined to leaflets 104-2, connector element 132 spanning a portion of the length of cords 120-2. In some embodiments, connector element 132 may be a piece of material surrounding or wrapped around cords 120-2 and sutured to cords 120-2. Connector element 132 may be shorter than cords 120-2 such that second end of cords 120-2 are free from each other and may be connected to different papillary muscles 124. In some embodiments, cords 120-2 may be between 2 and 10 mm in length, while connector element 132 may surround cords 120-2 over a length of between 1 and 5 mm. In some embodiments, the lengths of cords 120-2 and the length of cords 120-2 surrounded by connector element 132 are adjustable and may be determined by preoperative echo scans and CT scans performed on the patient receiving tricuspid valve prosthesis 100-2. In some embodiments, connector element 132 may be connected at one end to the tips 122-2 of leaflets 104-2 and on the distal end to a first end of the cords 120-2. In some embodiments, connector element 132 is connected to the leaflets 104-2 so as to enable sufficient coaptation when the edges of leaflets 104-2 are in contact one with the other. In some embodiments, connector element 132 may be sutured to tips 122-2 of leaflets 104-2. In some embodiments, connector element 132 is shaped as a hollow tube (not shown). In other embodiments, connector element 132 is shaped as a solid tube having an oval or circular cross section allowing for connecting the tips 122-2 of leaflets 104-2 and cords 120-2 thereto with minimal disruption to blood flow through the tricuspid valve prothesis 100-2. In some embodiments, connector element 132 length is between 1 and 5 mm. Connector element 132 ensures that there is a single vector of pulling when the heart contracts, even though cords 120-2 may be connected to different papillary muscles 124. Connector element 132 may advantageously ensure uniformity during coaptation so that coaptation of free edges 116-2 of tricuspid valve prothesis 100-2 is not compromised by the different locations of papillary muscles 124 or the differing distances to the papillary muscles 124.

Fig. 3C is a schematic illustration of a tricuspid valve prosthesis 100-3, which is another alternative embodiment of the tricuspid valve prosthesis 100 of Fig. 1, with a connector element 132-3, in accordance with embodiments of the present disclosure. The embodiment of Fig. 3C is similar to the embodiment of Fig. 1, with like-numbered terms configured in the same way, except that cords (not shown) are secured together with a connector element 132-3 spanning the entire length of cords (not shown). Cords (not shown) secured together with connector element 132-3 may then be stitched and secured to the papillary muscle of a patient, preferably the anterior papillary muscle, or a wall of a ventricle of the heart. Connector element 132-3 strengthens the connection between leaflets 104-3 and the papillary muscle or free wall of the right ventricle as connector element 132-3 brings cords 120-3 together to prevent tearing of cords 120-3 when the anterior papillary muscle pulls on cords 120-3 during heart contraction.

In other embodiments, connector element 132-3 may be connected at one end to the tips 122-3 of leaflets 104-3 and on the other end to a papillary muscle 124 of the patient, preferably the anterior papillary muscle, or a wall of a ventricle of the heart. In some embodiments, connector element 132-3 is sutured to tips 122-3 of leaflets 104-3. In some embodiments, connector element 132-3 is shaped as a hollow tube (not shown). In other embodiments, connector element 132-3 is shaped as a solid tube having an oval or circular cross section allowing for connecting the tips of leaflets 104-3 to a papillary muscle or a free wall of the right ventricle thereto with minimal disruption to blood flow through the tricuspid valve prothesis 100-3. Connector element 132-3 ensures that there is a single vector of pulling when the heart contracts. Connector element 132-3 may advantageously ensure uniformity during coaptation so that the coaptation of free edges 116-3 of tricuspid valve prosthesis 100-3 is not compromised during heart contraction.

Figs. 4A and 4B are schematic illustrations of methods of implanting tricuspid valve prosthesis 100-3 into a heart 200, in accordance with embodiments of the present disclosure. In some embodiments, cords 120-3 secured together with connector element 132-3 together as cap 128 in tricuspid valve prosthesis 100-3 may all be connected to the same papillary muscle 124 in the right ventricle (see Fig. 4A). In some embodiments, cords 120-3 secured together with connector element 132-3 may be connected to the anterior papillary muscle as it is the strongest papillary muscle. In some embodiments, cords 120-3 secured together with connector element 132-3 in tricuspid valve prosthesis 100-3 may be connected to a free wall 440 of right ventricle 212 (see Fig. 4B). In some embodiments, cords 120-3 secured together with connector element 132-3 in tricuspid valve prosthesis 100-3 may be connected to free wall 440 of right ventricle 212 by passing cords 120-3 secured together with connector element 132-3 through an incision (not shown) in free wall 440 of right ventricle 212 and stitching cords 120-3 secured together with connector element 132-3 to an inner surface 444 of free wall 440 of right ventricle 212 and an outer surface 448 of free wall 440 of right ventricle 212. In some embodiments, there may be an apical pad (not shown) placed at outer surface 448 of free wall 440 over the incision in free wall 440 of right ventricle 212. The methods of implanting tricuspid valve prosthesis 100-3 into heart 200 illustrated in Figs. 4A and 4B may similarly be employed to implant tricuspid valve prosthesis 100-1 with cords 120-1 stitched together into cap 128 into heart 200, or tricuspid valve prosthesis 100-3 with connector element 132-3 joined directly to tips 122-3 of leaflets 104-3. Joining cords 120 secured together with connector element 132 or cap 128, or joining connector element 132-3 directly to the papillary muscles 124 or free wall 440 of the right ventricle 212 provides support to the right ventricular wall throughout the cardiac cycle and prevent the leaflets 104 from opening into the right atrium 208. They also allow tricuspid valve prosthesis 100 to interact or "cross-talk" with the heart ventricle, thus advantageously reducing the risk of heart failure after implantation.

Fig. 5A is a schematic illustration of a side perspective view of leaflets 504 and ring 508 of a first alternative tricuspid valve prosthesis 500 and Fig. 5B is a schematic illustration of a bottom view of leaflets 504 of a first alternative tricuspid valve prosthesis 500, in accordance with embodiments of the present disclosure. First alternative tricuspid valve prosthesis 500 may be designed for a patient with a round-shaped annulus and a papillary muscle that is located close to a centre of the round-shaped annulus. As in tricuspid valve prosthesis 100, first alternative tricuspid valve prosthesis 500 has a ring 508. Ring 508 is circular to fit a round-shaped annulus of a patient. Ring 508 may have a diameter of between 20 and 35 mm to fit within a native tricuspid valve of a patient. Ring 408 may have a diameter corresponding to a native tricuspid valve annulus of a patient obtained through imaging of the patient's heart. As in tricuspid valve prosthesis 100, first alternative tricuspid valve prosthesis 500 has leaflets 504 suspended from ring 508, leaflets 504 connected to each other at commissures 512. First alternative tricuspid valve prosthesis 500 may have three leaflets: a first leaflet 504a, a second leaflet 504b, and a third leaflet 504c. First leaflet 504a may mimic a septal leaflet of a native tricuspid valve, second leaflet 504b may mimic a posterior leaflet of a native tricuspid valve, and third leaflet 504c may mimic an anterior leaflet of a native tricuspid valve. First leaflet 504a may be connected to second leaflet 504b at a first commissure 512a, second leaflet 504b may be connected to third leaflet 504c at a second commissure 512b, and third leaflet 504c may be connected to first leaflet 504a at a third commissure 512c. In some embodiments, first leaflet 504a, second leaflet 504b, and third leaflet 504c may be identical, with all three leaflets 504 having the same annular length 532. In other embodiments, first leaflet 504a, second leaflet 504b, and third leaflet 504c may be differing, with the three leaflets 504 having the differing annular lengths 532 (see Fig. 5C). In some embodiments, where ring 508 is round and first leaflet 504a, second leaflet 504b and third leaflet 504c are identical, first alternative tricuspid valve prosthesis 500 would be symmetrical and thus first alternative tricuspid valve prosthesis 500 may be orientated in any way during implantation.

Fig. 5C is a schematic illustration of a leaflet 504 and cord 520 of first alternative tricuspid valve prosthesis 500, in accordance with embodiments of the present disclosure. Tip 522 of each leaflet 504 may be connected to a first end 521 of a cord 520. Preferably, all three cords 520 are joined together to a single papillary muscle, and preferably the anterior papillary muscle. In some embodiments, a second end 523 of all three cords 520 may be secured together through any of the methods described above, including suturing cords 520 together to form a cap 128, or wrapping a connector element 132-3 around cords 520, and then secured to a single papillary muscle 124 (see Fig. 4A) or a free wall 440 of the right ventricle 212 (see Fig. 4B). In other embodiments, cords 520 may be stitched together or surrounded with a connector element 132 at a first end 521 connected to leaflets 504 and connected to separate papillary muscles 124 at a second end 523.

In some embodiments of the present disclosure, leaflet 504 may have an annular length 532 formed along a convex arc of 120° of a circle with a radius of between 20 and 40 mm. In some embodiments, annular length 532 may be 26.2 mm for a valve of size 25 mm, 28.3 mm for a valve of size 27 mm, 30.4 mm for a valve of size 29 mm, 32.4 mm for a valve of size 31 mm, and 34.5 mm for a valve of size 33 mm. Commissure 512 may have a length of between 3 and 10 mm. In some embodiments, free edge 516 may be formed along a concave arc of between 20° and 30° of a circle with a radius of between 30 and 60 mm. In some embodiments, cords 520 may have a length of between 5 and 15 mm, and a width of between 2 and 8 mm. In some embodiments, the dimensions of leaflet 504 may be determined based on the size of the native tricuspid valve of the patient and the vertical distance between the papillary muscle to the annulus (PM height). In some embodiments, first alternative tricuspid valve prosthesis 500 for a size 36 mm circular tricuspid valve with PM height of 30 mm or more may have three identical leaflets 504 with annular length 532 formed along an arc of 59.07° of a circle with a radius of 36.51, commissure 512 with a length of 10 mm, free edge 516 formed along an arc of 28° of a circle with radius 50 mm, and cords 520 with length of 8.62 mm and width of 1.46 mm. In some embodiments, leaflets 504a, 504b, and 504c may be dimensioned such that tip 522a of leaflet 504a, tip 522b of leaflet 504b and tip 522c of leaflet 504c converge at a point corresponding to a centre of ring 508 (see Fig. 5B). The various dimensions of leaflets 504 may be optimised and determined using finite element method (FEM) analysis to visualise the opening and closing of first alternative tricuspid valve prosthesis 500 under pressure.

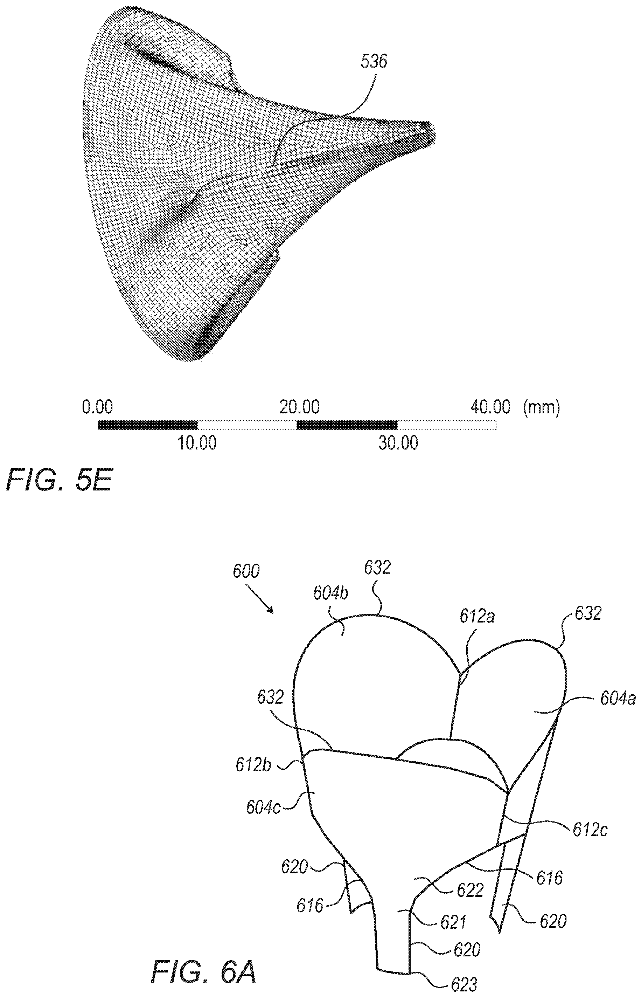

Fig. 5D is a schematic illustration of a top view of results of a finite element method (FEM) analysis of leaflets 504 of first alternative tricuspid valve prosthesis 500 under external pressure of 23 mmHg and Fig. 5E is a schematic illustration of a side view of results of a finite element method (FEM) analysis of leaflets 504 of first alternative tricuspid valve prosthesis 500 under maximum principal stress (MPa), in accordance with embodiments of the present disclosure. FEM analysis was carried out on leaflets 504 of first alternative tricuspid valve prosthesis 500 to visualise the opening and closing of tricuspid valve prosthesis 500 under pressure and identify areas of stress. Preferably, tricuspid valve prosthesis 500 closes at an external pressure of between 10 and 30 mmHg, which is the range of normal pulmonary artery pressure. The darker the area on the model, the higher the stress experienced by the area. The material used for FEM analysis may be bovine pericardium with a thickness of 0.28 mm and Young's modulus of 30M Pa, although other materials with other properties may be used. In some embodiments, thin and young age bovine pericardium with a low Young's modulus may be used. As illustrated in Figs. 5D and 5E, free edges 516 coapt with each other under pressure to form coaptation edges 536 and seal tricuspid valve prosthesis 500.

Fig. 6A is a schematic illustration of a side perspective view of a second alternative tricuspid valve prosthesis 600, in accordance with embodiments of the present disclosure. Fig. 6B is a schematic illustration of second alternative tricuspid valve prosthesis 600 in an open state, and Fig. 6C is a schematic illustration of second alternative tricuspid valve prosthesis 600 in a closed state, in accordance with embodiments of the present disclosure. Second alternative tricuspid valve prosthesis 600 is similar to first alternative tricuspid valve prosthesis 500 in that it has a circular annulus and comprises three leaflets: a first leaflet 604a, a second leaflet 604b, and a third leaflet 604c. In some embodiments, first leaflet 604a may mimic a septal leaflet of a native tricuspid valve, second leaflet 604b may mimic a posterior leaflet of a native tricuspid valve, and third leaflet 604c may mimic an anterior leaflet of a native tricuspid valve. First leaflet 604a may be connected to second leaflet 604b at a first commissure 612a, second leaflet 604b may be connected to third leaflet 604c at a second commissure 612b, and third leaflet 604c may be connected to first leaflet 604a at a third commissure 612c. First leaflet 604a, second leaflet 604b, and third leaflet 604c may be identical, with all three leaflets 604 having the same annular length 632. Annular lengths 632 of second alternative tricuspid valve prosthesis 600 may be sutured together along commissures 612 to form a ring 608 to be sutured onto a valve annulus 602 (see Fig. 6B). In some embodiments, annular length 632 may be 26.2 mm for a valve of size 25 mm, 28.3 mm for a valve of size 27 mm, 30.4 mm for a valve of size 29 mm, 32.4 mm for a valve of size 31 mm, and 34.5 mm for a valve of size 33 mm. In other embodiments, first leaflet 604a, second leaflet 604b, and third leaflet 604c may be different, with all three leaflets 604 having differing annular lengths 632.

In some embodiments, unlike first alternative tricuspid valve 500, second alternative tricuspid valve prosthesis 600 may be designed for surface-to-surface coaptation of leaflets 604 (see Fig. 6C). In some embodiments, in an open state (see Fig. 6B), the only points of contact between leaflets 604a, 604b and 604c are coaptation points 612a, 612b, and 612c, and free edges 616 of leaflets 604a, 604b and 604c do not contact each other. In some embodiments, in a closed state, leaflets 604 coapt with each other at a surface proximate to free edges 616, forming a coaptation surface 614 between leaflets 604. First leaflet 604a may coapt with second leaflet 604b to form coaptation surface 614a, second leaflet 604b may coapt with third leaflet 604c to form coaptation surface 614b, and third leaflet 604c may coapt with first leaflet 604a to form coaptation surface 614c. Such an embodiment has a higher coaptation surface 614 or area than the edge-to-edge coaptation in first alternative tricuspid valve 500. The area of coaptation surface 614 may be adjusted by altering the curvature of free edges 616.

In some embodiments, tip 622 of each leaflet 604 may be connected to a first end 621 of a cord 620. Preferably, all three cords 620 are joined together to a single papillary muscle 124, preferably the anterior papillary muscle. In some embodiments, a second end 623 of all three cords 620 may be secured together through any of the methods described above, including suturing cords 620 together to form a cap 128, or wrapping a connector element 132-3 around cords 620, and then secured to a single papillary muscle 124 (see Fig. 4A) or a free wall 440 of the right ventricle 212 (see Fig. 4B). In other embodiments, cords 620 may be stitched together or surrounded with a connector element 132 at first end 621 connected to leaflets 604 and connected to separate papillary muscles 124 at a second end 523.

Fig. 6D is a schematic illustration of an alternative cord 620-1 connected to leaflet 604 of second alternative tricuspid valve prosthesis 600, in accordance with embodiments of the present disclosure. As in cord 620, cord 620-1 may comprise a first end 621-1 and a second end 623-1. In some embodiments, first end 621-1 of cord 620-1 may split into two or more branches 625, with each branch 625 connected to leaflet 604. In some embodiments, by splitting cord 620-1 when connecting to leaflet 604, this may advantageously distribute the pressure effect to a different wall of the ventricle of the heart and may help long term durability of second alternative tricuspid valve prosthesis 600 and prevent heart failure.

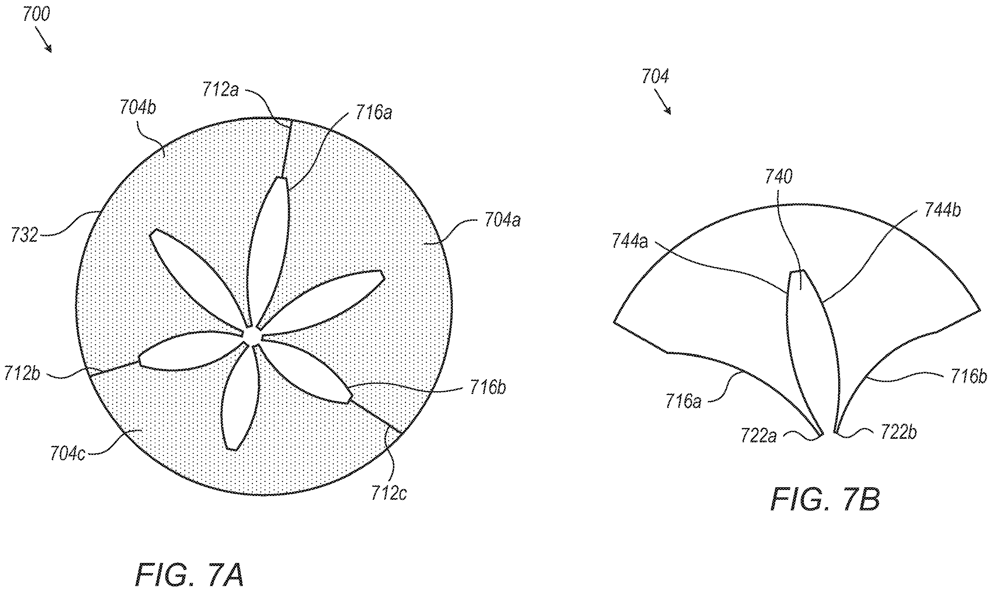

Fig. 7A is a schematic illustration of leaflets of a third alternative tricuspid valve prosthesis 700, in accordance with embodiments of the present disclosure. Third alternative tricuspid valve prosthesis 700 may be designed for a patient with a round-shaped annulus and a papillary muscle 124 that is located close to a centre of the round-shaped annulus. Third alternative tricuspid valve prosthesis 700 is similar to first alternative tricuspid valve prosthesis 500 in that it has a circular annulus and comprises three leaflets: a first leaflet 704a, a second leaflet 704b, and a third leaflet 704c. In some embodiments, first leaflet 704a may mimic a septal leaflet of a native tricuspid valve, second leaflet 704b may mimic a posterior leaflet of a native tricuspid valve, and third leaflet 704c may mimic an anterior leaflet of a native tricuspid valve. In some embodiments, first leaflet 704a may be connected to second leaflet 704b at a first commissure 712a, second leaflet 704b may be connected to third leaflet 704c at a second commissure 712b, and third leaflet 704c may be connected to first leaflet 704a at a third commissure 712c. First leaflet 704a, second leaflet 704b, and third leaflet 704c may be identical, with all three leaflets 704 having the same annular length 732. In some embodiments, annular length 732 may be 26.2 mm for a valve of size 25 mm, 28.3 mm for a valve of size 27 mm, 30.4 mm for a valve of size 29 mm, 32.4 mm for a valve of size 31 mm, and 34.5 mm for a valve of size 33 mm. In other embodiments, first leaflet 704a, second leaflet 704b, and third leaflet 704c may be different, with all three leaflets 704 having differing annular lengths 732.

In some embodiments of the present disclosure, leaflets 704 of third alternative tricuspid valve prosthesis 700 may each comprise two free edges 716 shaped in the form of concave arcs: a first free edge 716a, and a second free edge 716b. First free edge 716a and second free edge 716b may have the same dimensions or differing dimensions. In some embodiments, first free edge 716a and second free edge 716b may have the same dimensions and be shaped along a concave arc of 120° of a circle with a radius of between 20 and 35 mm. In some embodiments, first free edge 716a and second free edge 716b may be located on either side of leaflet 704.

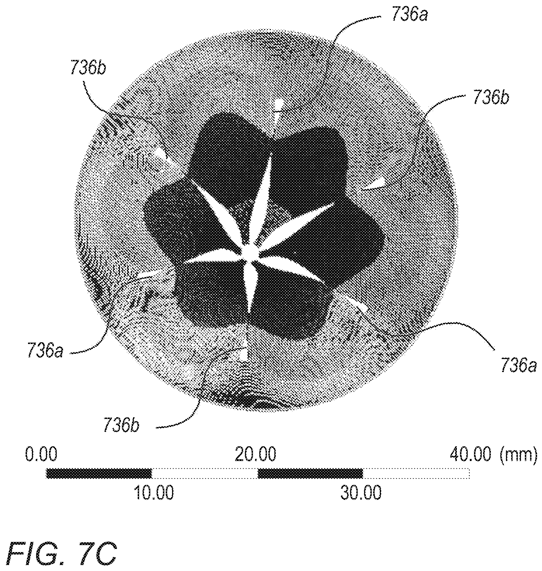

Fig. 7B is a schematic illustration of a leaflet 704 of third alternative tricuspid valve prosthesis 700, in accordance with embodiments of the present disclosure. Unlike first alternative tricuspid valve 500, each leaflet 704 of third alternative tricuspid valve prosthesis 700 is adapted to connect to two cords (not shown). Leaflet 704 may be connected to two cords by introducing a slot 740 into leaflet 704 which splits a tip 722 of leaflet 704 into two separate tips 722a and 722b. Slot 740 is shaped in the general shape of a biconvex lens bound by two circular arcs 744a and 744b, with an open vertex that splits tip 722 of leaflet 704. Slot 740 may be adapted such that the circular arcs 744a and 744b of slot 740 coapt under pressure to form coaptation edges 736b (see Fig. 7C). In some embodiments, circular arcs 744a and 744b of slot 740 may be shaped along an arc of between 20° and 30° of a circle with a radius of between 30 and 60 mm. Similar to first alternative tricuspid valve prosthesis 500, tips 722a and 722b may each be connected to a first end of a cord (not shown), the second end of the cords connected to papillary muscles with methods similar to those described in relation to first alternative tricuspid valve prosthesis 500. The various dimensions of leaflets 704 may be optimised and determined using finite element method (FEM) analysis to visualise the opening and closing of tricuspid valve prosthesis under pressure. Third alternative tricuspid valve prosthesis 700 has a larger effective orifice area (EOA) or valve opening area than first alternative tricuspid valve prosthesis 500 due to the addition of slots 740 into leaflet 704, which may advantageously lead to increased hydrodynamic performance. EOA is a critical metric of valve function such that the larger the EOA, the lower the pressure gradient across the valve. A lower or reduced pressure gradient may reduce wear and tear of the valve and increase durability of the valve as well as improved heart recovery. The patient may suffer less heart failure and better performance.

Fig. 7C is a schematic illustration of a top view of results of a finite element method (FEM) analysis of leaflets 704 of third alternative tricuspid valve prosthesis 700 under external pressure of 23 mmHg, in accordance with embodiments of the present disclosure. FEM analysis was carried out on leaflets 704 of third alternative tricuspid valve prosthesis 700 to visualise the opening and closing of third alternative tricuspid valve prosthesis 700 under pressure and identify areas of stress. The darker the area on the model, the higher the stress experienced by the area. The material used for FEM analysis may be bovine pericardium with a thickness of 0.28 mm and Young's modulus of 30 MPa, although other materials with other properties may be used. As illustrated in Fig. 7C, free edges 716 coapt with each other under external pressure to form coaptation edges 736a, and circular arcs 744a and 744b coapt with each other under external pressure to firm coaptation edges 736b to seal third alternative tricuspid valve prosthesis 700.

Fig. 8A is a schematic illustration of leaflets of a fourth alternative tricuspid valve prosthesis 800, in accordance with embodiments of the present disclosure. Fourth alternative tricuspid valve prosthesis 800 may be designed for a patient with a papillary muscle that is located closer to the tricuspid valve annulus edge rather than a centre of the annulus. Fourth alternative tricuspid valve prosthesis 800 has a bean-shaped annulus ring which is a closed elongated curved shaped with a concave arc-shaped indentation on a first longitudinal length. Fourth alternative tricuspid valve prosthesis 800 may comprise two leaflets 804 to achieve good valve closing. Fourth alternative tricuspid valve prosthesis 800 may comprise a first leaflet 804a and a second leaflet 804b. In some embodiments, first leaflet 804 may mimic the function of a septal leaflet of a native tricuspid valve, while second leaflet 804b may mimic the function of a posterior leaflet and an anterior leaflet of a native tricuspid valve. First leaflet 804a may be connected to second leaflet 804b at a first commissure 812a and a second commissure 812b. In some embodiments, first commissure 812a may correspond with the posteroseptal commissure of a native tricuspid valve, and second commissure 812b may correspond with the anteroseptal commissure of a native tricuspid valve. In some embodiments, the first commissure 812a or second commissure 812b may correspond with the anterioposteror commissure of a native tricuspid valve, or any other suitable position may not correspond to a commissure of a native tricuspid valve. In some embodiments, the positions of the first commissure 812a and second commissure 812b may be determined based on a location of the papillary muscles of the heart. First commissure 812a and second commissure 812b may have lengths of between 3 and 10 mm. First commissure 812a and second commissure 812b may have the same length, or may have different lengths. In some embodiments, annular length 832 of first leaflet 804a may include the concave arc-shaped indentation on the first longitudinal length. First leaflet 804a may have a first annular length of 832a and second leaflet 804b may have a second annular length of 832b. In some embodiments, a tricuspid valve prosthesis 800 with a standard size of 25 mm may have a first annular length 832a of 39.0 mm and a second annular length 832b of 45.6 mm. In some embodiments, a tricuspid valve prosthesis 800 with a standard size of 27 mm may have a first annular length 832a of 42.2 mm and a second annular length 832b of 49.3 mm. In some embodiments, a tricuspid valve prosthesis 800 with a standard size of 29 mm may have a first annular length 832a of 45.3 mm and a second annular length 832b of 52.9 mm. In some embodiments, a tricuspid valve prosthesis 800 with a standard size of 31 mm may have a first annular length 832a of 48.4 mm and a second annular length 832b of 56.6 mm. In some embodiments, a tricuspid valve prosthesis 800 with a standard size of 33 mm may have a first annular length 832a of 51.5 mm and a second annular length 832b of 60.3 mm.

In some embodiments of the present disclosure, first leaflet 804a may comprise a first free edge 816a and a second free edge 816b. Second leaflet 804a may comprise a third free edge 816c and a fourth free edge 816d. In a closed state, first free edge 816a of first leaflet 804a may coapt with third free edge 816c of second leaflet 804b to form first coaptation edge 836a (see Fig. 8B), while second free edge 816b of first leaflet 804b may coapt with fourth free edge 816d of second leaflet to form coaptation edge 836b (see Fig. 8B). First free edge 816a and third free edge 816c may have corresponding dimensions while second free edge 816b and fourth free edge 816d may have corresponding dimensions. In some embodiments, first free edge 816a and third free edge 816c may be formed along an arc of 50° of a circle with radius 21 mm, while second free edge 816b and fourth free edge 816d may be formed along an arc of 50° of a circle with radius 28 mm.

Fig. 8B is a schematic illustration of a top view of results of a finite element method (FEM) analysis of leaflets 804 of fourth alternative tricuspid valve prosthesis 800 under external pressure of 23 mmHg, in accordance with embodiments of the present disclosure. FEM analysis was carried out on leaflets 804 of fourth alternative tricuspid valve prosthesis 800 to visualise the opening and closing of tricuspid valve prosthesis 800 under pressure and identify areas of stress. The darker the area on the model, the higher the stress experienced by the area. The material used for FEM analysis may be bovine pericardium with a thickness of 0.28 mm and Young's modulus of 30 MPa, although other materials with other properties may be used. As illustrated in Figs. 8B, free edges 816 coapt with each other under external pressure to form coaptation edges 836 and seal fourth alternative tricuspid valve prosthesis 800.

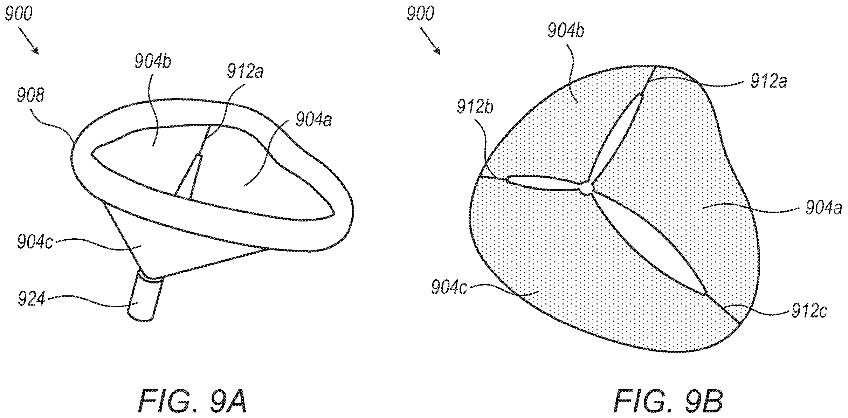

Fig. 9A is a schematic illustration of a side perspective view of a fifth alternative tricuspid valve prosthesis 900 designed for a type I tricuspid valve and Fig. 9B is a schematic illustration of a top view of leaflets of fifth alternative tricuspid valve prosthesis 900 designed for a type I tricuspid valve, in accordance with embodiments of the present disclosure. 54% of patients have type I tricuspid valve morphology, which is the most prevalent tricuspid valve morphology. A type I tricuspid valve has three leaflets: a septal leaflet, a posterior leaflet, and an anterior leaflet. In addition, the anterior papillary muscle of a type I tricuspid valve is located slightly offset towards the posterior leaflet and anterior leaflet. Similar to a type I tricuspid valve, fifth alternative tricuspid valve prosthesis 900 designed for a type I tricuspid valve has three leaflets 904: a first leaflet 904a mimicking the septal leaflet of a type I native tricuspid valve, a second leaflet 904b mimicking the posterior leaflet of a type I native tricuspid valve, and a third leaflet 904c mimicking the anterior leaflet of a type I native tricuspid valve. First leaflet 904a may be connected to second leaflet 904b at a first commissure 912a, second leaflet 904b may be connected to third leaflet 904c at a second commissure 912b, and third leaflet 904c may be connected to first leaflet 904a at a third commissure 912c. In some embodiments, first commissure 912a may correspond with the posteroseptal commissure of a native tricuspid valve, second commissure 912b may correspond with the anteroposterior commissure of a native tricuspid valve, and third commissure 912c may correspond with the anteroseptal commissure of a native tricuspid valve. In some embodiments, commissures 912 may be labelled with laser or with any other appropriate method to assist with orientating fifth alternative tricuspid valve prosthesis 900 during implantation

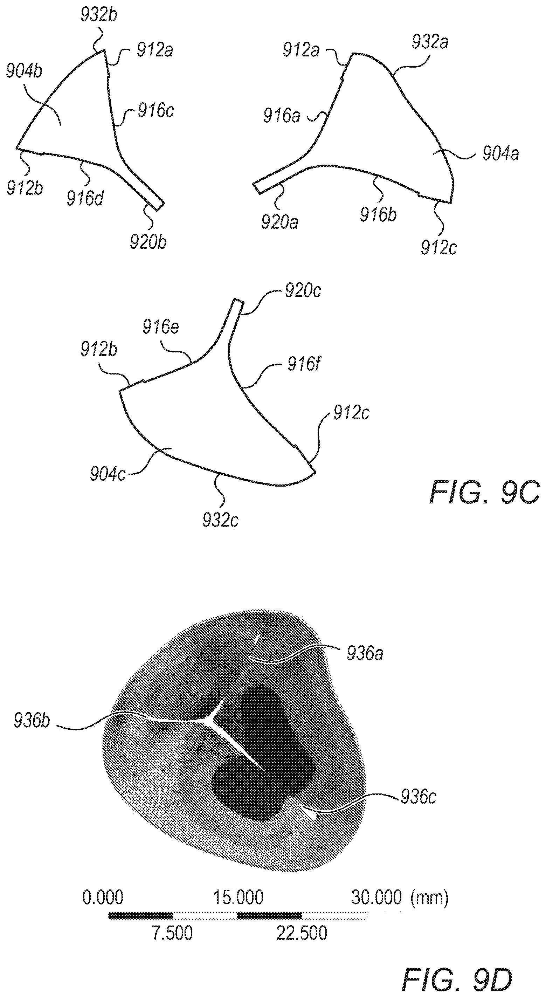

Fig. 9C is a schematic illustration of leaflets 904 and cords 920 of fifth alternative tricuspid valve prosthesis 900, in accordance with embodiments of the present disclosure. First leaflet 904a may have a first annular length 932a, second leaflet 904b may have a second annular length 932b, while third leaflet 904c may have an annular length 932c. Annular lengths 932 of each leaflet 904 and positions of commissures 912 may be adjusted or personalised based on measurements taken of a patient's native tricuspid valve and annulus. Alternatively, annular lengths 932 may be adjusted based on a valve standard size. In some embodiments, a valve with standard size 25 mm may have a first annular length 932a of 27.9 mm, a second annular length 932b of 19.4 mm, and a third annular length 932c of 34.5 mm. In some embodiments, a valve with standard size 27 mm may have a first annular length 932a of 30.1 mm, a second annular length 932b of 21.0 mm, and a third annular length 932c of 37.2 mm. In some embodiments, a valve with standard size 29 mm may have a first annular length 932a of 32.4 mm, a second annular length 932b of 22.5 mm, and a third annular length 932c of 40.0 mm. In some embodiments, a valve with standard size 31 mm may have a first annular length 932a of 34.4 mm, a second annular length 932b of 24.1 mm, and a third annular length 932c of 42.8 mm. In some embodiments, a valve with standard size 33 mm may have a first annular length 932a of 36.8 mm, a second annular length 932b of 25.6 mm, and a third annular length 932c of 45.5 mm.

In some embodiments of the present disclosure, first leaflet 904a may have a first free edge 916a and a second free edge 916b. Second leaflet 904b may have a third free edge 916c and a fourth free edge 916d. Third leaflet 904c may have a fifth free edge 916e and a sixth free edge 916f. Preferably, first free edge 916a and third free edge 916c have corresponding dimensions, fourth free edge 916d and fifth free edge 916e have corresponding dimensions, and sixth free edge 916f and second free edge 916b have corresponding dimensions such that when fifth alternative tricuspid valve prosthesis 900 is in a closed state, first free edge 916a of first leaflet 904a may coapt with third free edge 916c of second leaflet 904b to form coaptation edge 936a, fourth free edge 916d of second leaflet 904b may coapt with fifth free edge 916e of third leaflet 904c to form coaptation edge 936b, and sixth free edge 916f of third leaflet 604c may coapt with second free edge 916b of first leaflet 904a to form coaptation edge 936c (see Fig. 9D). In some embodiments, first free edge 916a and third free edge 916c may be formed along a concave arc of between 50° and 70° of a circle with a radius of between 12 and 18 mm. In some embodiments, fourth free edge 916d and fifth free edge 916e may be formed along a concave arc of between 50° and 70° of a circle with a radius of between 10 and 15 mm. In some embodiments, sixth free edge 916f and second free edge 916b may be formed along a concave arc of between 30° and 50° of a circle with a radius of between 25 and 35 mm. The various dimensions of leaflets 904 may be optimised and determined using finite element method (FEM) analysis to visualise the opening and closing of tricuspid valve prosthesis under pressure. Preferably, leaflets 904a, 904b and 904c are dimensioned such that the tips of the leaflets converge at a point above a single papillary muscle 124, preferably the anterior papillary muscle.

In some embodiments of the present disclosure, first leaflet 904a may be connected to a first end of a first cord 920a, second leaflet 904b may be connected to a first end of a second cord 920b, and third leaflet 904c may be connected to a first end of a third cord 920c. In some embodiments, first cord 920a may have a length of between 5 and 15 mm, and a width of between 2 and 5 mm. In some embodiments, second cord 920b may have a length of between 5 and 15 mm, and a width of between 2 and 5 mm. In some embodiments, third cord 920c may have a length of between 5 and 15 mm, and a width of between 2 and 5 mm. Preferably, cords 920a, 920b and 920c have the same lengths and are connected to a single papillary muscle 124, and preferably the anterior papillary muscle, or free wall 440 of right ventricle 212 through any methods disclosed in Figs. 3A to 3C, 4A and 4B. Cords 920a, 920b and 920c may be sutured together to form a cap or be surrounded by a connector element 924 before suturing to the single papillary muscle or free wall.

Fig. 9D is a schematic illustration of a top view of results of a finite element method (FEM) analysis of leaflets 904 of fifth alternative tricuspid valve prosthesis 900 under external pressure of 23 mmHg, in accordance with embodiments of the present disclosure. The material used for FEM analysis may be bovine pericardium with a thickness of 0.28 mm and Young's modulus of 30 MPa, although other materials with other properties may be used. As illustrated in Figs. 9D, free edges 916 coapt with each other under external pressure to form coaptation edges 936 and seal fifth alternative tricuspid valve prosthesis 900.

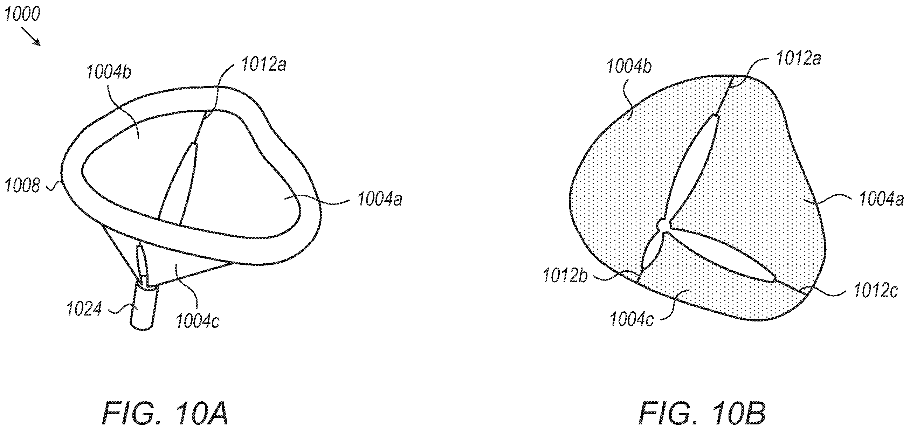

Fig. 10A is a schematic illustration of a side perspective view of a sixth alternative tricuspid valve prosthesis 1000 designed for a type IIIB tricuspid valve and Fig. 10B is a schematic illustration of a top view of leaflets of sixth alternative tricuspid valve prosthesis 1000 designed for a type IIIB tricuspid valve, in accordance with embodiments of the present disclosure. 32% of patients have type IIIB tricuspid valve morphology, which is the second-most prevalent tricuspid valve morphology. A type IIIB tricuspid valve has four leaflets: a septal leaflet, two posterior leaflets, and an anterior leaflet. The anterior papillary muscle of a type IIIB tricuspid valve is located slightly offset towards the posterior leaflet and anterior leaflet. On the other hand, sixth alternative tricuspid valve prosthesis 1000 designed for a type IIIB tricuspid valve has three leaflets 1004: a first leaflet 1004a mimicking the septal leaflet of a type IIIB native tricuspid valve, a second leaflet 1004b mimicking the two posterior leaflets of a type IIIB native tricuspid valve, and a third leaflet 1004c mimicking the anterior leaflet of a type IIIB native tricuspid valve. First leaflet 1004a may be connected to second leaflet 1004b at a first commissure 1012a, second leaflet 1004b may be connected to third leaflet 1004c at a second commissure 1012b, and third leaflet 1004c may be connected to first leaflet 1004a at a third commissure 1012c. In some embodiments, first commissure 1012a may correspond with the posteroseptal commissure of a native tricuspid valve, second commissure 1012b may correspond with the anteroposterior commissure of a native tricuspid valve, and third commissure 1012c may correspond with the anteroseptal commissure of a native tricuspid valve. In some embodiments, commissures 1012 may be labelled with laser or with any other appropriate method to assist with orientating sixth alternative tricuspid valve prosthesis 1000 during implantation

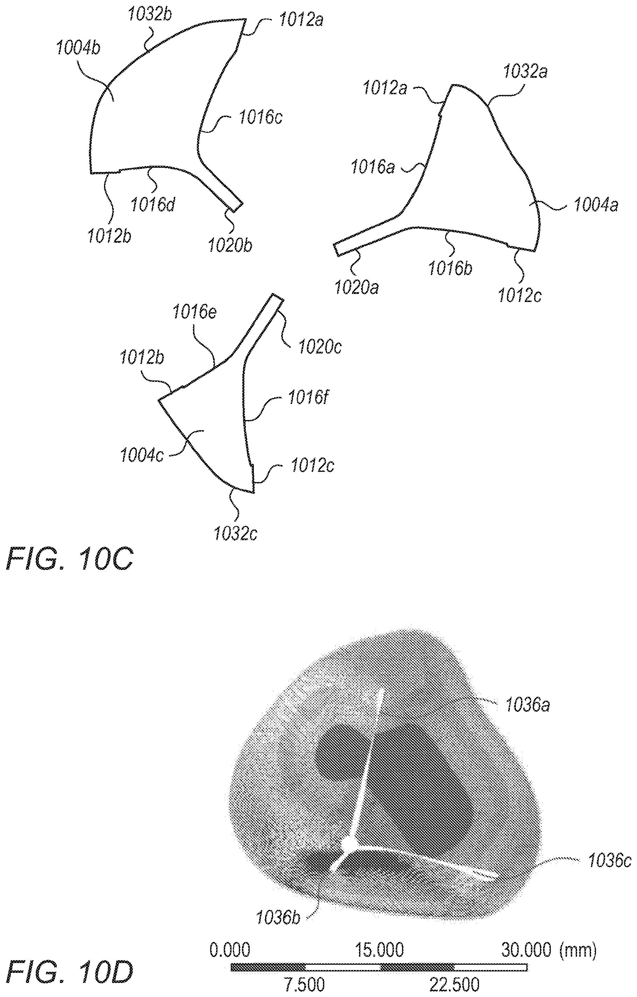

Fig. 10C is a schematic illustration of leaflets 1004 and cords 1020 of sixth alternative tricuspid valve prosthesis 1000, in accordance with embodiments of the present disclosure. First leaflet 1004a may have a first annular length 1032a, second leaflet 1004b may have a second annular length 1032b, while third leaflet 1004c may have an annular length 1032c. In some embodiments, annular lengths 1032 of each leaflet 1004 and positions of commissures 1012 may be adjusted or personalised based on measurements taken of a patient's native tricuspid valve and annulus. Alternatively, annular lengths 1032 may be adjusted based on a valve standard size. In some embodiments, a valve with standard size 25 mm may have a first annular length 1032a of 27.6 mm, a second annular length 832b of 34.4 mm, and a third annular length 1032c of 18.9 mm. In some embodiments, a valve with standard size 27 mm may have a first annular length 1032a of 29.8 mm, a second annular length 1032b of 37.1 mm, and a third annular length 1032c of 20.4 mm. In some embodiments, a valve with standard size 29 mm may have a first annular length 1032a of 32.0 mm, a second annular length 1032b of 39.9 mm, and a third annular length 1032c of 21.9 mm. In some embodiments, a valve with standard size 31 mm may have a first annular length 1032a of 34.2 mm, a second annular length 1032b of 42.6 mm, and a third annular length 1032c of34.2 mm. In some embodiments, a valve with standard size 33 mm may have a first annular length 1032a of 36.4 mm, a second annular length 1032b of 45.3 mm, and a third annular length 1032c of 24.9 mm.

In some embodiments of the present disclosure, first leaflet 1004a may have a first free edge 1016a and a second free edge 1016b. In some embodiments, second leaflet 1004b may have a third free edge 1016c and a fourth free edge 1016d. In some embodiments, third leaflet 1004c may have a fifth free edge 1016e and a sixth free edge 1016f. Preferably, first free edge 1016a and third free edge 1016c have corresponding dimensions, fourth free edge 1016d and fifth free edge 1016e have corresponding dimensions, and sixth free edge 1016f and second free edge 1016b have corresponding dimensions such that when sixth alternative tricuspid valve prosthesis 1000 is in a closed state, first free edge 1016a of first leaflet 1004a may coapt with third free edge 1016c of second leaflet 1004b to form coaptation edge 1036a, fourth free edge 816d of second leaflet 1004b may coapt with fifth free edge 1016e of third leaflet 1004c to form coaptation edge 1036b, and sixth free edge 1016f of third leaflet 1004c may coapt with second free edge 1016b of first leaflet 1004a to form coaptation edge 1036c (see Fig. 10D). In some embodiments, first free edge 1016a and third free edge 1016c may be formed along a concave arc of between 30° and 50° of a circle with a radius of between 25 and 30 mm. Fourth free edge 1016d and fifth free edge 1016e may be formed along a concave arc of between 50° and 70° of a circle with a radius of between 10 and 15 mm. Sixth free edge 1016f and second free edge 1016b may be formed along a concave arc of between 50° and 70° of a circle with a radius of between 15 and 25 mm. In some embodiments, the various dimensions of leaflets 1004 may be optimised and determined using finite element method (FEM) analysis to visualise the opening and closing of tricuspid valve prosthesis under pressure. Preferably, leaflets 1004a, 1004b and 1004c are dimensioned such that the tips of the leaflets converge at a point above a single papillary muscle, preferably the anterior papillary muscle. In other embodiments, leaflets 1004 may be connected to the single papillary muscle or free wall though a connector element connected to tips of leaflets 1004.

In some embodiments of the present disclosure, first leaflet 1004a may be connected to a first end of a first cord 1020a, second leaflet 1004b may be connected to a first end of a second cord 1020b, and third leaflet 1004c may be connected to a first end of a third cord 1020c. In some embodiments, first cord 1020a may have a length of between 5 and 15 mm, and a width of between 2 and 5 mm. Second cord 1020b may have a length of between 5 and 15 mm, and a width of between 2 and 5 mm. Third cord 1020c may have a length of between 5 and 15 mm, and a width of between 2 and 5 mm. Preferably, cords 1020a, 1020b and 1020c have the same lengths and are connected to a single papillary muscle 124, and preferably the anterior papillary muscle, or free wall 440 of right ventricle 212 through any methods disclosed in Figs. 3A to 3C, 4A and 4B. Cords 1020a, 1020b and 1020c may be sutured together to form a cap or be surrounded by a connector element 1024 before suturing to the single papillary muscle or free wall.

Fig. 10D is a schematic illustration of a top view of results of a finite element method (FEM) analysis of leaflets 1004 of sixth alternative tricuspid valve prosthesis 1000 under external pressure of 23 mmHg, in accordance with embodiments of the present disclosure. The material used for FEM analysis may be bovine pericardium with a thickness of 0.28 mm and Young's modulus of 30 MPa, although other materials with other properties may be used. As illustrated in Fig. 10D, free edges 1016 coapt with each other under external pressure to form coaptation edges 1036 and seal sixth alternative tricuspid valve prosthesis 1000.

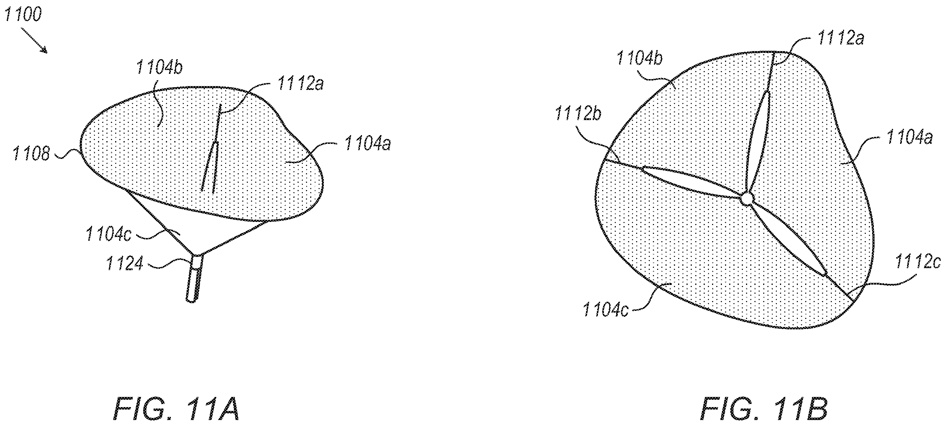

Fig. 11A is a schematic illustration of a side perspective view of a seventh alternative tricuspid valve prosthesis 1100 and Fig. 11B is a schematic illustration of a top view of leaflets of seventh alternative tricuspid valve prosthesis 1100, in accordance with embodiments of the present disclosure. Seventh alternative valve prosthesis 1100 is not designed specifically for any native tricuspid valve morphology. Seventh alternative tricuspid valve prosthesis 1100 has three leaflets 1104: a first leaflet 1104a mimicking the septal leaflet of a native tricuspid valve, a second leaflet 1104b mimicking posterior leaflet of a native tricuspid valve, and a third leaflet 1104c mimicking the anterior leaflet of a native tricuspid valve. First leaflet 1104a may be connected to second leaflet 1104b at a first commissure 1112a, second leaflet 1104b may be connected to third leaflet 1104c at a second commissure 1112b, and third leaflet 1104c may be connected to first leaflet 1104a at a third commissure 1112c. In some embodiments, first commissure 1112a may correspond with the posteroseptal commissure of a native tricuspid valve, second commissure 1112b may correspond with the anteroposterior commissure of a native tricuspid valve, and third commissure 1112c may correspond with the anteroseptal commissure of a native tricuspid valve. In some embodiments, commissures 1112 may be labelled with laser or with any other appropriate method to assist with orientating seventh alternative tricuspid valve prosthesis 1100 during implantation.

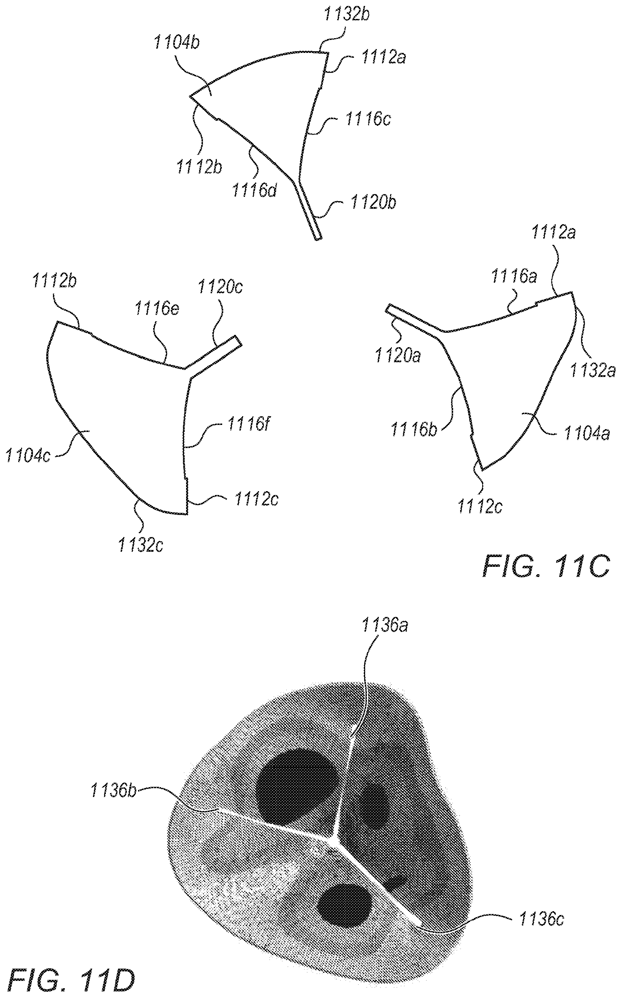

Fig. 11C is a schematic illustration of leaflets 1104 and cords 1120 of seventh alternative tricuspid valve prosthesis 1100, in accordance with embodiments of the present disclosure. First leaflet 1104a may have a first annular length 1132a, second leaflet 1104b may have a second annular length 1132b, while third leaflet 1104c may have an annular length 1132c. In some embodiments, annular lengths 1132 of each leaflet 1104 and positions of commissures 1112 may be adjusted or personalised based on measurements taken of a patient's native tricuspid valve and annulus. Alternatively, annular lengths 1132 may be adjusted based on a valve standard size. In some embodiments, a valve with standard size 25 mm may have a first annular length 1132a of 27.7 mm, a second annular length 832b of 19.7 mm, and a third annular length 1132c of 34.5 mm. In some embodiments, a valve with standard size 27 mm may have a first annular length 1132a of 30.0 mm, a second annular length 1132b of 21.3 mm, and a third annular length 1132c of 37.3 mm. In some embodiments, a valve with standard size 29 mm may have a first annular length 1132a of 32.2 mm, a second annular length 1132b of 22.8 mm, and a third annular length 1132c of 40.0 mm. In some embodiments, a valve with standard size 31 mm may have a first annular length 1132a of 34.4 mm, a second annular length 1132b of 24.4 mm, and a third annular length 1132c of 42.8 mm. In some embodiments, a valve with standard size 33 mm may have a first annular length 1132a of 36.6 mm, a second annular length 1132b of 26.0 mm, and a third annular length 1132c of 45.6 mm.

In some embodiments of the present disclosure, first leaflet 1104a may have a first free edge 1116a and a second free edge 1116b. Second leaflet 1104b may have a third free edge 1116c and a fourth free edge 1116d. Third leaflet 1104c may have a fifth free edge 1116e and a sixth free edge 1116f. Preferably, first free edge 1116a and third free edge 1116c have corresponding dimensions, fourth free edge 1116d and fifth free edge 1116e have corresponding dimensions, and sixth free edge 1116f and second free edge 1116b have corresponding dimensions such that when seventh alternative tricuspid valve prosthesis 1100 is in a closed state, first free edge 1116a of first leaflet 1104a may coapt with third free edge 1116c of second leaflet 1104b to form coaptation edge 1136a, fourth free edge 1116d of second leaflet 1104b may coapt with fifth free edge 1116e of third leaflet 1104c to form coaptation edge 1136b, and sixth free edge 1116f of third leaflet 1104c may coapt with second free edge 1116b of first leaflet 1104a to form coaptation edge 1136c (see Fig. 11D). In some embodiments, first free edge 1116a and third free edge 1116c may be formed along a concave arc of between 50° and 70° of a circle with a radius of between 12 and 20 mm. In some embodiments, fourth free edge 1116d and fifth free edge 1116e may be formed along a concave arc of between 50° and 70° of a circle with a radius of between 12 and 20 mm. In some embodiments, sixth free edge 1116f and second free edge 1116d may be formed along a concave arc of between 50° and 70° of a circle with a radius of between 12 and 18 mm. In some embodiments, the various dimensions of leaflets 1104 may be optimised and determined using finite element method (FEM) analysis to visualise the opening and closing of tricuspid valve prosthesis under pressure. Preferably, leaflets 1104a, 1104b and 1104c are dimensioned such that the tips of the leaflets converge at a point corresponding to a centre of the native tricuspid valve annulus.

In some embodiments of the present disclosure, first leaflet 1104a may be connected to a first end of a first cord 1120a, second leaflet 1104b may be connected to a first end of a second cord 1120b, and third leaflet 1104c may be connected to a first end of a third cord 1120c. Preferably, first end of cords 1120a, 1120b, and 1120c connected together either through suturing or with a connector element 1124 and second end of cords 1120a, 1120b, and 1120c are free and connected to different papillary muscles. In some embodiments, second end of first cord 1120a may be connected to the septal papillary muscle, second end of second cord 1120b may be connected to a posterior papillary muscle, and second end of third cord 1120c may be connected to an anterior papillary muscle. In some embodiments, cords 1120a, 1120b, and 1120c may have differing lengths and thickness from each other as they are connected to different papillary muscles which would have different distances from the tips of leaflets 1104a, 1104b and 1100c. In some embodiments, first cord 1120a may have a length of between 5 and 15 mm, and a width of between 2 and 5 mm. Second cord 1120b may have a length of between 5 and 15 mm, and a width of between 2 and 5 mm. Third cord 1120c may have a length of between 5 and 15 mm, and a width of between 2 and 5 mm.

Fig. 11D is a schematic illustration of a top view of results of a finite element method (FEM) analysis of leaflets 1104 of seventh alternative tricuspid valve prosthesis 1100 under external pressure of 23 mmHg, in accordance with embodiments of the present disclosure. The material used for FEM analysis may be bovine pericardium with a thickness of 0.28 mm and Young's modulus of 30 MPa, although other materials with other properties may be used. As illustrated in Figs. 11D, free edges 1116 coapt with each other under external pressure to form coaptation edges 1136 and seal seventh alternative tricuspid valve prosthesis 1100.

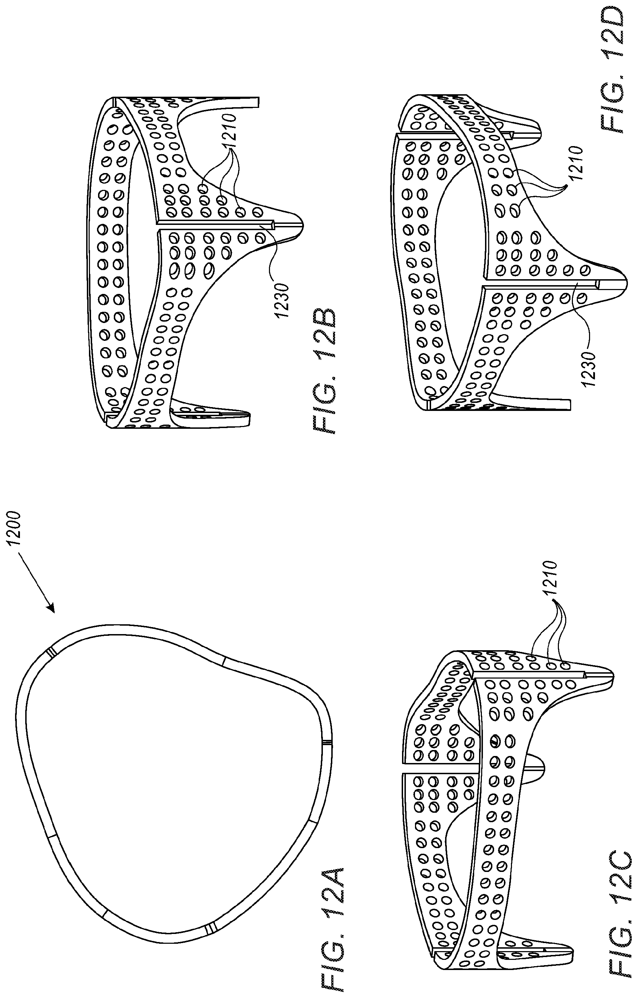

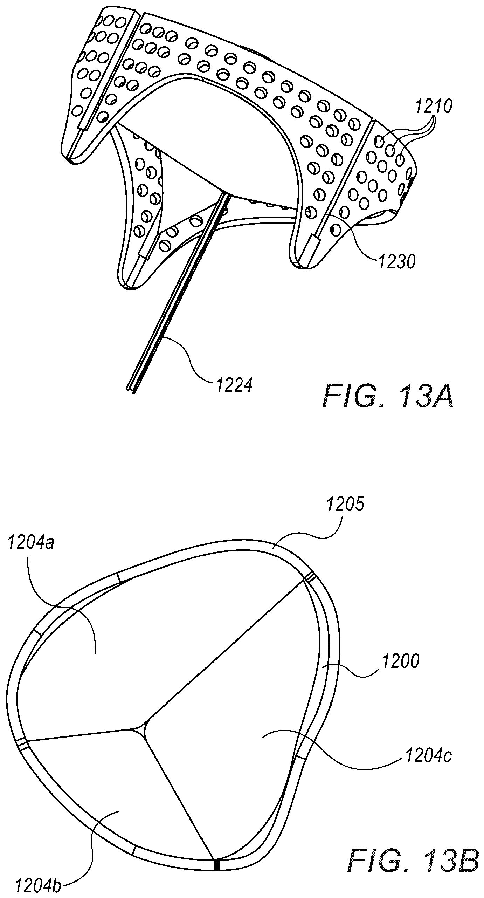

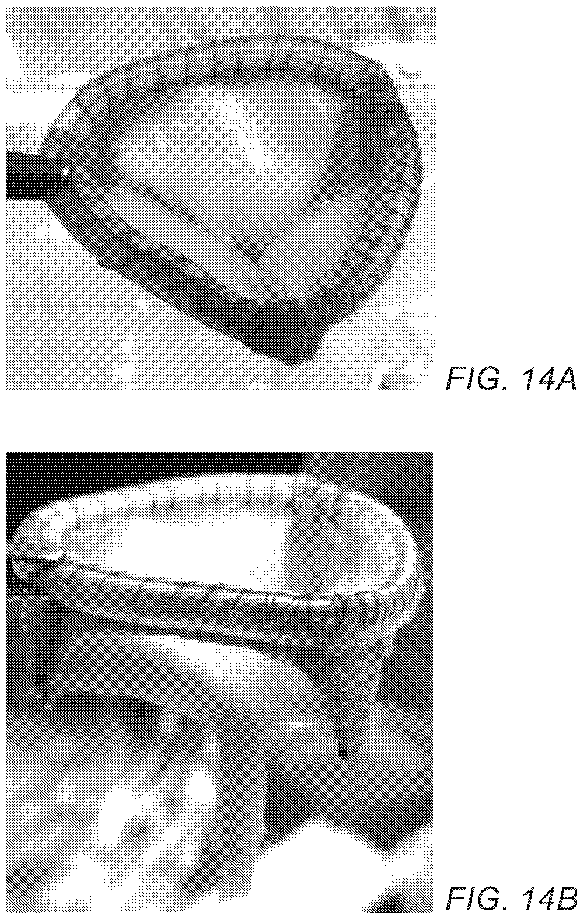

In some embodiments, the tricuspid valve prosthesis of the present disclosure may comprise a stent frame 1200. It is therefore to be understood that unless specifically stated, when the tricuspid valve prosthesis disclosed herein does not include a stent frame, the tricuspid valve prosthesis is considered as a non-stented tricuspid valve prosthesis such as those described in Figs. 1, and 3A-3C. However, it is to be appreciated that whenever applicable, in addition to the embodiment that will be described below, the various embodiments of the tricuspid valve prosthesis including the first, second, third, fourth, fifth, sixth and seventh alternative tricuspid valve prostheses (refer to Figs. 5A-E, 6A-D, 7A-C, 8A-B, 9A-D, 10A-D, and 1 1A-D) described above may also include a stent frame. Figs. 12A-12D illustrate stent 1200 that may be included in the tricuspid valve prosthesis. As used herein, the terms "stent" and "stent frame" may be used interchangeably and they have the same meaning. Stent 1200 may advantageously be used as a support for the tricuspid valve prosthesis. In such an embodiment, the tricuspid valve prosthesis may be termed as stented tricuspid valve prosthesis. Tricuspid valve prosthesis provided with a stent frame may advantageously allow ease of retrieval of the valve prosthesis. The perimeter of stent frame 1200 may be adapted to mimic the shape of the native tricuspid valve annulus. In some embodiments, the perimeter may be a circle or an irregular circle (such as a bean-shaped ring). Fig. 12A is an exemplary embodiment of the perimeter of stent frame 1200 having irregular circular shape that mimics the shape of the native tricuspid valve annulus. Stent 1200 may be made of stainless steel, silicon, plastic, graphene oxide or other suitable materials. In some embodiments, stent 1200 may be made of medical grade materials such as those the U.S. Pharmacopeial Convention (USP) Class VI certified or compliant materials or materials complying with ISO 10993. In some embodiments, a cuff 1205 may be attached to stent 1200 (see Fig. 13B). It is therefore to be understood that cuff 1205 may be dimensioned to match the perimeter of stent 1200. According to some embodiments of the present disclosure, cuff 1205 may be a fabric cuff band 1205 attached to stent 1200 and is advantageously used to strengthen the ring formed by the pericardium and to stitch the valve with annulus muscle (see Figs. 14A and 14B). As can be appreciated, in some embodiments, cuff 1205 may be considered as a strengthening mechanism for the ring. In some embodiments, cuff band 1205 may comprise silicon and a radiopaque wire embedded. One or more drugs may be coated on fabric cuff 1205 or wire to prevent or minimize pannus formation, calcification or combination thereof.

In some embodiments, stent 1200 may be designed according to the location of the papillary muscles. As can be seen from at least Figs. 12B-12D, in some embodiments, stent 1200 may comprise a plurality of holes 1210 for stitching of the pericardium and one or more slots 1230 for the leaflets (not shown) to be inserted to. In some embodiments, when stent 1200 is made of silicon or other materials of similar properties, stent 1200 may be provided without the plurality of holes. Each hole 1210 of the plurality of holes may have a diameter in a range from about 0.5 mm to about 2 mm, preferably of 1 mm. In some embodiments, the diameter of the hole in the plurality of holes may be uniform. In some embodiments, each hole of the plurality of holes may have different diameter ranging from about 0.5 mm to about 2 mm. In some embodiments, the placement or configuration of the plurality of holes 1210 may be adjusted accordingly. In an exemplary embodiment, each hole of the plurality of holes may be spaced in similar distance with the neighbouring or adjacent holes. Each slot of the one or more slots 1230 may have a length from about 8 mm to about 16 mm. In some embodiments, when the valve size is of 29 mm, a suitable length of the slot may be 12 mm. Accordingly, the length of slot 1230 may be adjusted depending on the valve size. In some embodiments, stent frame 1200 may have a thickness from about 0.5 mm to about 2 mm, preferably 1 mm. In some embodiments, for a valve size of 29 mm, stent 1200 may have a perimeter of about 100.81 mm and a height of about 15 mm. It is to be understood that such parameters including perimeter and height may be adjusted according to the valve size. Hence, generally stent 1200 may have a perimeter in a range from about 80 mm to about 140 mm. In some embodiments, stent 1200 may have a height in a range from about 12 mm to about 25 mm.

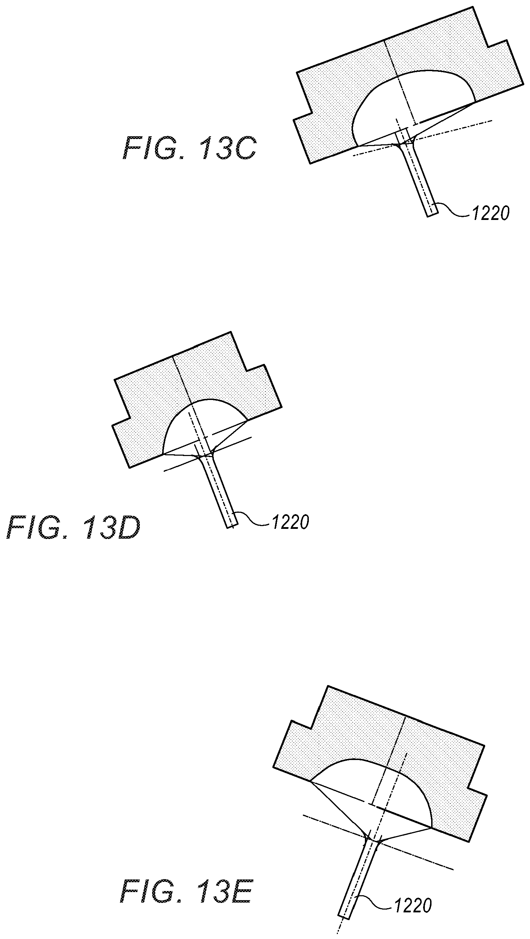

Figs. 13C-13E illustrate the three leaflets i.e. anterior leaflet 1204c (Fig. 13C), posterior leaflet 1204b (Fig. 13D), and septal leaflet 1204a (Fig. 13E) of the stented tricuspid valve described in Figs. 13A and 13B, respectively, but prior to mounting these leaflets to stent frame 1200. As shown in Figs. 13C-13E, once the leaflets are mounted to stent frame 1200, each of the leaflets may have three-sided shape as previously discussed. In other words, prior to assembling these leaflets, the leaflets or pericardium may comprise extended portions (see the greyed area in Figs. 13C-13D) used for securing the pericardium to stent frame 1200. In securing the pericardium to stent frame 1200, the extended portion may be folded. As can be seen from Fig. 13A, cords 1220 of the leaflets may be sutured together to form a cap or be surrounded by a connector element 1224 before suturing to the single papillary muscle or free wall. Fig. 13B illustrates stented tricuspid valve prosthesis having stent 1200, fabric cuff band 1205, septal leaflet 1204a, posterior leaflet 1204b and anterior leaflet 1204c. For a tricuspid valve prosthesis comprising a stent, the height of each of the leaflets with cords may be in a range from about 30 mm to 55 mm (see Figs. 13C-13E). In some embodiments, the height measured may include the extended portions mentioned above. In some embodiments, for a valve size of 29 mm, the height of each of the leaflets with cords may be about 43 mm.

As illustrated by Figs. 14A and 14B, the tricuspid valve prosthesis with a stent frame may be manufactured by shaping the leaflets by cutting out the pericardium (human or bovine pericardium), followed by stitching the shaped leaflets onto the stent to form the tricuspid valve prosthesis having a stent frame. Prior to stitching the leaflets, the shaped leaflets having the extended portions can be mounted to the frame by folding the extended portions of the leaflets. The folding may substantially enclose the one or more slots of the stent frame. Optionally, the folding may also include attaching a cuff to the frame followed by stitching the leaflets. The shaped leaflets may be anterior leaflet, posterior leaflet and septal leaflet. In such an embodiment, the leaflets (anterior leaflet, posterior leaflet and septal leaflet) may be positioned within the stent frame.

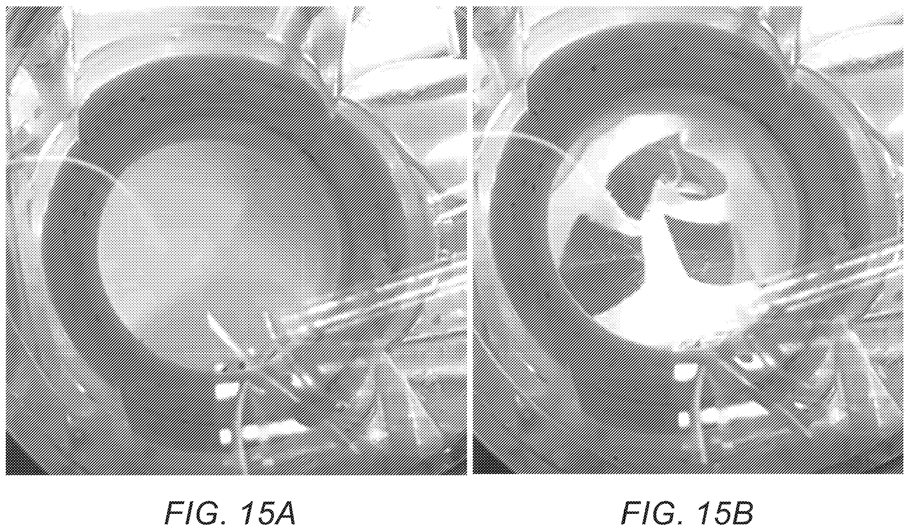

Figs. 15A and 15B illustrate top view of the stented tricuspid valve prosthesis in accordance with embodiments of the present disclosure. During hydrodynamic testing, such as systole and diastole in vitro hydrodynamic testing, the stented tricuspid valve prosthesis may close (Fig. 15A) and open (Fig. 15B). Similar to stentless tricuspid valve prosthesis described above, in the stented tricuspid valve, the leaflets may coapt with each other to form coaptation surfaces or coaptation edges and seal the valve prosthesis. Advantageously, such arrangement may minimize leakage during deployment of the tricuspid valve prosthesis described herein.

It should be appreciated that the above-described methods and apparatus may be varied in many ways, including omitting, or adding steps, changing the order of steps and the type of devices used. It should be appreciated that different features may be combined in different ways. In particular, not all the features shown above in a particular embodiment are necessary in every embodiment of the disclosure. Further combinations of the above features are also considered to be within the scope of some embodiments of the disclosure.

It will be appreciated by persons skilled in the art that the present invention is not limited to what has been particularly shown and described hereinabove. Rather the scope of the present invention is defined only by the claims, which follow.

## Claims

1. A tricuspid valve prosthesis (100) to be transplanted into a heart, the tricuspid valve prosthesis (100) comprising:
two or more leaflets (104) each comprising an annular length (532) and two free edges (116) forming a tip (122), the two or more leaflets (104) joined together at commissures (112) to create a ring (108), and configured to coapt with each other;
two or more sets of cords (120), each set of cords (120) attached to the tip (122) of one of the two or more leaflets (104) at a first end (121);
**characterized in that** the tricuspid valve prosthesis (100) comprises a single connector element (132) directly connected to each of the tips (122) of the two or more leaflets (104), such that that the two or more sets of cords (120) are surrounded by the single connector element (132).

2. The tricuspid valve prosthesis (100) of claim 1, wherein the single connector element (132) is configured to be attached to a papillary muscle of the heart and wherein the papillary muscle is an anterior papillary muscle.

3. The tricuspid valve prosthesis (100) of claim 1, wherein the single connector element (132) is configured to be attached to a free wall of a right ventricle.

4. The tricuspid valve prosthesis (100) of claim 1, further comprising two or more sets of cords, each set of cords attached to the single connector element (132) at a first end (121).

5. The tricuspid valve prosthesis (100) of claim 1, wherein the single connector element (132) surrounds a first end (122) of the two or more sets of cords (120) and spans the length of the two or more sets of cords (120).

6. The tricuspid valve prosthesis (100) of claim 1 or 4, wherein the two or more sets of cords (120) are configured to be attached to one or more papillary muscles of the heart at a second end.

7. The tricuspid valve prosthesis (100) of claim 1, wherein the ring is bean-shaped or saddle-shaped and the free edges are shaped along a concave arc of a circle.

8. The tricuspid valve prosthesis (100) of claim 1, wherein the two or more leaflets (104) further comprise a slot (740) that splits the tip (122) of the two or more leaflets (104), the slot (740) comprising two circular arcs (744a, 744b), the two circular arcs (744a, 744b) are configured to coapt with each other.

9. The tricuspid valve prosthesis (100) of claim 1, further comprising a stent frame (1200), the stent frame (1200) comprising:
one or more slots (1230) for inserting the two or more leaflets (104) to the stent frame (1200);
a plurality of holes (1210) for stitching of the two or more leaflets (104); and
a cuff (1205) coated with one or more drugs and attached to the stent frame (1200);
wherein two or more leaflets (104) are positioned within the stent frame (1200); and wherein the ring (108) is dimensioned to match perimeter of the stent frame (1200).

## Patentansprüche

1. Trikuspidalklappenprothese (100) zur Implantation in ein Herz, wobei die Trikuspidalklappenprothese (100) umfasst:
zwei oder mehr Klappensegel (104), die jeweils einen ringförmigen Abschnitt (532) und zwei freie Ränder (116) aufweisen, die eine Spitze (122) bilden, wobei die zwei oder mehr Klappensegel (104) an den Kommissuren (112) miteinander verbunden sind, um einen Ring (108) zu bilden, und so ausgebildet sind, dass sie aneinander anliegen;
zwei oder mehr Sätze von Schnüren (120), wobei jeder Satz von Schnüren (120) an einem ersten Ende (121) an der Spitze (122) eines der zwei oder mehr Segel (104) befestigt ist;
**dadurch gekennzeichnet, dass** die Trikuspidalklappenprothese (100) ein einziges Verbindungselement (132) umfasst, das direkt mit jeder der Spitzen (122) der zwei oder mehr Klappensegel (104) verbunden ist, sodass die zwei oder mehr Sätze von Schnüren (120) von dem einzigen Verbindungselement (132) umgeben sind.

2. Die Trikuspidalklappenprothese (100) nach Anspruch 1, wobei das einzelne Verbindungselement (132) so ausgebildet ist, dass es an einem Papillarmuskel des Herzens befestigt werden kann, und wobei es sich bei dem Papillarmuskel um einen vorderen Papillarmuskel handelt.

3. Die Trikuspidalklappenprothese (100) nach Anspruch 1, wobei das einzelne Verbindungselement (132) so ausgebildet ist, dass es an einer freien Wand eines rechten Ventrikels befestigt werden kann.

4. Die Trikuspidalklappenprothese (100) nach Anspruch 1, die ferner zwei oder mehr Sätze von Schnüren umfasst, wobei jeder Satz von Schnüren an einem ersten Ende (121) an dem einzelnen Verbindungselement (132) befestigt ist.

5. Die Trikuspidalklappenprothese (100) nach Anspruch 1, wobei das einzelne Verbindungselement (132) ein erstes Ende (122) der zwei oder mehr Sätze von Schnüren (120) umgibt und sich über die Länge der zwei oder mehr Sätze von Schnüren (120) erstreckt.

6. Die Trikuspidalklappenprothese (100) nach Anspruch 1 oder 4, wobei die zwei oder mehr Sätze von Schnüren (120) so ausgebildet sind, dass sie an einem zweiten Ende an einem oder mehreren Papillarmuskeln des Herzens befestigt werden können.

7. Die Trikuspidalklappenprothese (100) nach Anspruch 1, wobei der Ring bohnenförmig oder sattelförmig ist und die freien Ränder entlang eines konkaven Kreisbogens geformt sind.

8. Die Trikuspidalklappenprothese (100) nach Anspruch 1, wobei die zwei oder mehr Klappensegel (104) ferner einen Schlitz (740) aufweisen, der die Spitze (122) der zwei oder mehr Klappensegel (104) teilt, wobei der Schlitz (740) zwei Kreisbögen (744a, 744b) umfasst und die beiden Kreisbögen (744a, 744b) so ausgebildet sind, dass sie aneinander anliegen.

9. Die Trikuspidalklappenprothese (100) nach Anspruch 1, die ferner einen Stentrahmen (1200) umfasst, wobei der Stentrahmen (1200) umfasst:
einen oder mehrere Schlitze (1230) zum Einführen der zwei oder mehr Klappensegel (104) in den Stentrahmen (1200);
eine Vielzahl von Löchern (1210) zum Vernähen der zwei oder mehr Klappensegel (104); und
eine Manschette (1205), die mit einem oder mehreren Wirkstoffen beschichtet und am Stentrahmen (1200) befestigt ist;
wobei zwei oder mehr Klappen (104) innerhalb des Stentrahmens (1200) angeordnet sind; und wobei der Ring (108) so dimensioniert ist, dass er dem Umfang des Stentrahmens (1200) entspricht.

## Revendications

1. Prothèse de valve tricuspide (100) destinée à être transplantée dans un cœur, la prothèse de valve tricuspide (100) comprenant :
deux ou plusieurs feuillets (104) comprenant chacun une longueur annulaire (532) et deux bords libres (116) formant une pointe (122), les deux ou plusieurs feuillets (104) étant joints ensemble aux commissures (112) pour créer un anneau (108), et configurés pour se coapter l'un à l'autre ;
deux ou plusieurs ensembles de cordons (120), chaque ensemble de cordons (120) étant attaché à la pointe (122) de l'un des deux ou plusieurs feuillets (104) à une première extrémité (121) ;
**caractérisée en ce que** la prothèse de valve tricuspide (100) comprend un élément de connexion unique (132) directement connecté à chacune des pointes (122) des deux ou plusieurs feuillets (104), de sorte que les deux ou plusieurs ensembles de cordons (120) soient entourés par l'élément de connexion unique (132).

2. Prothèse de valve tricuspide (100) selon la revendication 1, dans laquelle l'élément de connexion unique (132) est configuré pour être fixé à un muscle papillaire du cœur et dans laquelle le muscle papillaire est un muscle papillaire antérieur.

3. Prothèse de valve tricuspide (100) selon la revendication 1, dans laquelle l'élément de connexion unique (132) est configuré pour être fixé à une paroi libre d'un ventricule droit.

4. Prothèse de valve tricuspide (100) selon la revendication 1, comprenant en outre deux ou plusieurs ensembles de cordons, chaque ensemble de cordons étant fixé à l'élément de connexion unique (132) à une première extrémité (121).

5. Prothèse de valve tricuspide (100) selon la revendication 1, dans laquelle l'élément de connexion unique (132) entoure une première extrémité (122) des deux ou plusieurs ensembles de cordons (120) et couvre la longueur des deux ou plusieurs ensembles de cordons (120).

6. Prothèse de valve tricuspide (100) selon la revendication 1 ou 4, dans laquelle les deux ou plusieurs ensembles de cordons (120) sont configurés pour être fixés à un ou plusieurs muscles papillaires du cœur à une seconde extrémité.

7. Prothèse de valve tricuspide (100) selon la revendication 1, dans laquelle l'anneau est en forme de haricot ou de selle et les bords libres sont formés le long d'un arc de cercle concave.

8. Prothèse de valve tricuspide (100) selon la revendication 1, dans laquelle les deux ou plusieurs feuillets (104) comprennent en outre une fente (740) qui divise la pointe (122) des deux ou plusieurs feuillets (104), la fente (740) comprenant deux arcs circulaires (744a, 744b), les deux arcs circulaires (744a, 744b) étant configurés pour se coapter l'un à l'autre.

9. Prothèse de valve tricuspide (100) selon la revendication 1, comprenant en outre une armature de stent (1200), l'armature de stent (1200) comprenant :
une ou plusieurs fentes (1230) pour insérer les deux ou plusieurs feuillets (104) dans l'armature de stent (1200) ;
une pluralité de trous (1210) pour la couture des deux ou plusieurs feuillets (104) ; et
un manchon (1205) recouvert d'un ou plusieurs médicaments et fixé à l'armature de stent (1200) ;
dans lequel deux ou plusieurs feuillets (104) sont positionnés à l'intérieur de l'armature de stent (1200) ; et
dans lequel l'anneau (108) est dimensionné pour correspondre au périmètre de l'armature de stent (1200).
